# EUROPÄISCHE PATENTSCHRIFT

(11) **EP 2 630 198 B1**
(45) Veröffentlichungstag und Bekanntmachung des Hinweises auf die Patenterteilung: **19.08.2015**
(21) Anmeldenummer: 11723920.2
(22) Anmeldetag: 30.05.2011
(51) Int. Cl.: C09B 44/16, C09B 44/18

(54) **DIKATIONISCHE 4-AZA-1-AZONIABICYCLO[2.2.2]OCTANE UND MITTEL ZUM FÄRBEN VON KERATINHALTIGEN FASERN**
DICATIONIC 4-AZA-1-AZONIABICYCLO[2.2.2]OCTANES AND AGENTS FOR COLOURING KERATIN-CONTAINING FIBRES
4-AZA-1-AZONIABICYCLO[2.2.2]OCTANE DICATIONIQUE ET AGENT DE COLORATION DE FIBRES CONTENANT DE LA KÉRATINE

(30) Priorität: 20.10.2010 DE 102010042696
(43) Veröffentlichungstag der Anmeldung: 28.08.2013
(73) Patentinhaber: Henkel AG & Co. KGaA, 40589 Düsseldorf (DE)
(72) Erfinder: GROSS, Wibke, 41836 Hückelhoven (DE); GIESA, Helmut, 40670 Meerbusch (DE); KROOS, Astrid, 40789 Monheim (DE)
(86) Internationale Anmeldenummer: PCT/EP2011/058791
(87) Internationale Veröffentlichungsnummer: WO 2012/052194

(56) Entgegenhaltungen:
- EP-A2- 1 408 919
- WO-A1-02/078658
- WO-A2-2007/144280
- US-A1- 2006 037 151

## Beschreibung

Die Erfindung betrifft neue kationische Farbstoffe sowie Mittel zum Färben und gegebenenfalls gleichzeitigen Aufhellen von keratinhaltigen Fasern, insbesondere menschlichen Haaren, die diese Farbstoffe enthalten, die Verwendung dieser kationischen Farbstoffe zum Färben von Haaren sowie ein Verfahren zum Färben von keratinhaltigen Fasern, insbesondere menschlichen Haaren. Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraamino-pyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin, 2,5,6-Triamino-4-hydroxy-pyrimidin und 4,5-Diamino-1-(2-hydroxyethyl)pyrazol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O_{2,} was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Ein Vorteil bei der Haarfärbung mit direktziehenden Farbstoffen liegt vor allem in den leuchtenden Modenuancen, die mit dieser Färbemethode erzielt werden können. Bei Verbrauchern mit dunklerem Haarfarbton muß jedoch zeitgleich mit der Färbung in Modenuancen eine Blondierung stattfinden, welche die natürliche Haarfarbe durch Zerstörung der haareigenen Melaninpigmente aufhellt und damit den eingesetzten Farbstoffen mehr Brillanz verleiht. Für eine leichte Aufhellung genügt der Einsatz von Wasserstoffperoxid allein, für die stärkere Aufhellung über mehrere Nuancen wird jedoch üblicherweise eine Kombination aus Wasserstoffperoxid und Persulfatsalzen eingesetzt. Bislang sind dem Fachmann aus dem Stand der Technik kaum brillante Farbstoffe bekannt, die eine ausreichende Stabilität gegenüber Wasserstoffperoxid / Persulfatsalzen aufweisen und gleichzeitig gute anwendungstechnische Eigenschaften besitzen.

Es ist daher die Aufgabe dieser Erfindung, neuartige direktziehende Farbstoffe bereit zu stellen, welche die Haare in besonders brillanten Nuancen, insbesondere Rotnuancen, färben. Zudem sollten diese neuartigen Direktzieher nicht sensibilisierend sein und gute Echtheitseigenschaften besitzen. Der Fokus der erfindungsgemäßen Aufgabe liegt insbesondere im Auffinden von neuartigen Direktziehern, die eine hohe Stabilität gegenüber Oxidationsmitteln wie Wasserstoffperoxid und insbesondere der Kombination aus Wasserstoffperoxid und Persulfatsalzen besitzen. Darüber hinaus sollten die Direktzieher auch bei anderen wichtigen Echtheitseigenschaften wie beispielsweise Waschechtheiten, Schweißechtheiten und Reibechtheiten ein gutes Anforderungsprofil besitzen.

Es konnte nun in nicht vorhersehbarer Weise gefunden werden, dass Verbindungen der allgemeinen Formel (I) sich sehr gut zur intensiven Färbung von Haaren in leuchtenden Rotnuancen eignen. Es war überraschend, dass die erfindungsgemäßen Verbindungen nicht nur eine gute Oxidationsstabilität, sondern ebenfalls herausragende Eigenschaften im Hinblick auf ihre Waschechtheit, Schweißechtheit und Reibechtheit besitzen.

Färbemittel, enthaltend Verbindungen gemäß nachstehender Formel (I) sowie einige der entsprechenden Verbindungen selbst sind bislang nicht bekannt.

Gegenstand der vorliegenden Erfindung sind Mittel zum Färben und gegebenenfalls gleichzeitigen Aufhellen von Keratinfasern, insbesondere menschlichen Haaren, enthaltend mindestens einen dikationischen Azofarbstoff der Formel (I): 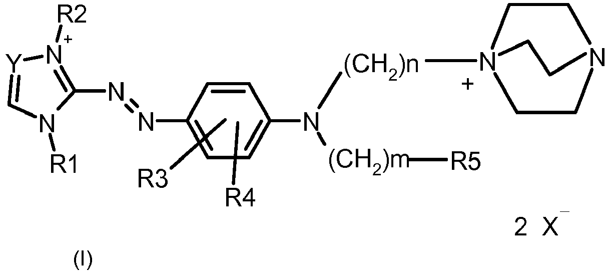 in der
- Y: steht entweder für =CH- oder ein Stickstoffatom,
- R¹, R²: stehen unabhängig voneinander für eine C₁-C₆-Alkylgruppe, eine C₂-C₆-Alkenylgruppe, eine Hydroxy-C₁-C₆-alkylgruppe, eine Polyhydroxy-C₂-C₆-alkylgruppe, eine C₁-C₆-Alkoxy-C₂-C₆-alkylgruppe, eine Cyano-C₁-C₆-alkylgruppe, eine Arylgruppe oder eine Aryl-C₁-C₆-alkylgruppe,
- R³, R⁴: stehen unabhängig voneinander für ein Wasserstoffatom, eine C₁-C₆-Alkylgruppe, ein Halogenatom (Fluor, Chlor, Brom oder lod), eine C₁-C₆-Alkoxygruppe, eine Aminogruppe, eine C₁-C₆-Alkylaminogruppe, eine Di-C₁-C₆-alkylaminogruppe, eine Hydroxygruppe oder aber R³ und R⁴ bilden zusammen einen 5-,6- oder 7-gliedrigen, gesättigten oder unsgesättigten Ring, der gegebenenfalls weitere Heteroatome enthalten oder weitere Substituenten tragen kann kann,
- n,m: stehen unabhängig voneinander für eine ganze Zahl von 1 bis 6,
- R⁵: steht für ein Wasserstoffatom, eine C₁-C₆-Alkoxygruppe, ein Halogenatom (Fluor, Chlor, Brom oder lod), eine Hydroxygruppe, eine C₁-C₆-Alkoxy-C₂-C₆-alkyloxygruppe, eine Nitrilgruppe, eine Arylgruppe oder eine Heteroarylgruppe,
- X-: steht für ein einwertiges Anion, vorzugsweise für Halogenid, Hydrogensulfat, ½ Sulfat, Benzolsulfonat, p-Toluolsulfonat, Acetat, Citrat, Lactat, Tetrafluoroborat, Trifluormethansulfonat, Hexafluorophosphat.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose verwendet werden. Der erfindungsgemäß verwendete Begriff "Färben von Keratinfasern" umfasst jedwede Form der Farbveränderung der Fasern. Umfasst sind insbesondere die unter den Begriffen Tönung, Blondierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise Blondierungen.

Beispiele für (C₁ bis C₆)-Alkylreste sind die Gruppen Methyl, Ethyl, Propyl, Isopropyl, n-Butyl, sec-Butyl und tert.-Butyl, n-Pentyl und n-Hexyl. Propyl, Ethyl und Methyl sind bevorzugte Alkylreste. Beispiele für entsprechende cyclische Alkylgruppen sind Cyclopentyl und Cyclohexyl.

Beispiele für bevorzugte (C₂ bis C₆)-Alkenylreste sind Vinyl und Allyl.

Weiterhin können als bevorzugte Beispiele für eine (C₁ bis C₆)-Monohydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyethylgruppe genannt werden. Eine 2-Hydroxyethylgruppe ist besonders bevorzugt.

Beispiele für eine (C₂ bis C₆)-Polyhydroxyalkylgruppe sind die 2,3-Dihydroxypropylgruppe, 3,4-Dihydroxybutylgruppe und die 2,4-Dihydroxybutylgruppe.

Erfindungsgemäß bevorzugte (C₁ bis C₆)-Alkoxygruppen sind beispielsweise eine Methoxy- oder eine Ethoxygruppe.

Die Methoxyethyl-, Ethoxyethyl-, Methoxypropyl-, Methoxybutyl-, Ethoxybutyl- und die Methoxyhexylgruppe sind Beispiele für erfindungsgemäße (C₁ bis C₆)-Alkoxy-(C₂ bis C₆)-alkylgruppen.

Eine bevorzugte Hydroxy-(C₁-C₆)-alkoxygruppe ist die 2-Hydroxyethoxygruppe.

Beispiele für Halogenatome sind F-, Cl-, Br- oder I-Atome, wobei Br- oder Cl-Atome ganz besonders bevorzugt sind.

Die Aminomethyl-, 2-Aminoethyl-, 3-Aminopropyl-, 2-Dimethylaminoethyl-, Diethylaminomethyl-, Dimethylaminomethyl, 2-Methylaminoethyl, Dimethylamino, 1-Piperidinomethyl, 1-Pyrrolidinomethyl, 4-Morpholinomethyl, Bis(2-hydroxyethyl)amino und die Aminogruppe sind Beispiele für eine Gruppe R⁵R⁶N-(CH₂)ₙ-, wobei die Diethylaminomethyl-, 1-Piperidinomethyl, 2-Dimethylaminoethyl-, Dimethylamino- und die Aminogruppe besonders bevorzugt sind.

Im Zuge der zu dieser Erfindung führenden Arbeiten hat es sich als besonders vorteilhaft herausgestellt, wenn die Reste R¹ und R² unabhängig voneinander für eine C₁-C₆-Alkylgruppe, eine C₂-C₆-Alkenylgruppe, eine Cyano-C₁-C₆-alkylgruppe oder eine Aryl-C₁-C₆-alkylgruppe stehen. Insbesondere bevorzugt ist es, wenn R¹ und R² beide für eine C₁-C₆-Alkylgruppe (insbesondere eine Methyl oder eine Ethylgruppe), eine C₂-C₆-Alkenylgruppe (insbesondere eine Allylgruppe) oder eine Aryl-C₁-C₆-alkylgruppe (insbesondere eine Benzylgruppe) stehen.

Erfindungsgemäße Mittel zum Färben und gegebenenfalls gleichzeitigen Aufhellen keratinischer Fasern, die Verbindungen der Formel (I) enthalten, in der die Reste R¹ und R² für eine C₁-C₆-Alkylgruppe (insbesondere eine Methyl- oder Ethylgruppe), eine C₂-C₆-Alkenylgruppe (insbesondere eine Allylgruppe) oder eine Aryl-C₁-C₆-alkylgruppe (insbesondere eine Benzylgruppe) stehen, sind bevorzugte Ausführungsformen der vorliegenden Erfindung.

Weiterhin sind Verbindungen der allgemeinen Formel (I), bei denen R³ und R⁴ unabhängig voneinander für ein Wasserstoffatom, eine C₁-C₆-Alkylgruppe, ein Halogenatom (Fluor, Chlor, Brom oder lod) oder eine C₁-C₆-Alkoxygruppe stehen, zur Erfüllung der erfindungsgemäßen Aufgabenstellung besonders geeignet. Insbesondere bevorzugt sind hierbei Verbindungen, bei denen sowohl R³ als auch für R⁴ für ein Wasserstoffatom stehen.

n steht bevorzugt für die Zahlen 2 oder 3,

m steht bevorzugt für die Zahlen 1, 2 oder 3.

Wenn R⁵ für ein Wasserstoffatom, eine C₁-C₆-Alkoxygruppe, eine Nitrilgruppe oder eine Arylgruppe steht, ist dies ebenfalls bevorzugt. Ganz besonders bevorzugt sind in diesem Zusammenhang Verbindungen der allgemeinen Formel (I), bei denen R⁵ für ein Wasserstoffatom steht.

Bei den erfindungsgemäßen Verbindungen der allgemeinen Formel (I) handelt es sich um dikationische Verbindungen, deren positive Ladungen durch eine entsprechende Anzahl negativer Ladungen des Gegenions X neutralisiert werden. Die Neutralisation kann durch das Vorhandensein von zwei einfach negativ geladenen Gegenionen erfolgen (2 X⁻), aber auch die Neutralisation durch ein doppelt negativ geladenes Gegenion (X²⁻) ist erfindungsgemäß.

Im Rahmen einer ersten bevorzugten Ausführungsform steht Y für ein Stickstoffatom. Innerhalb dieser ersten bevorzugten Ausführungsform enthalten besonders bevorzugte Mittel zum Färben und gegebenenfalls Aufhellen von Haaren mindestens eine Verbindung ausgewählt aus der Gruppe der
- Salze des 1,4-Dimethyl-5-[(4-{methyl[2-(4-aza-1-azoniabicyclo[2.2.2]octan-1-yl)ethyl]amino}phenyl)diazenyl]-4H-1,2,4-triazol-1-iums
- Salze des 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salze des 1,4-Dimethyl-5-[(4-{propyl[2-(4-aza-1-azoniabicyclo[2.2.2]octan-1-yl)ethyl]amino}phenyl)diazenyl]-4H-1,2,4-triazol-1-iums
- Salze des 1,4-Diethyl-5-[(4-{methyl[2-(4-aza-1-azoniabicyclo[2.2.2]octan-1-yl)ethyl]amino}phenyl)diazenyl]-4H-1,2,4-triazol-1-iums
- Salze des 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salze des 1,4-Diethyl-5-[(4-{propyl[2-(4-aza-1-azoniabicyclo[2.2.2]octan-1-yl)ethyl]amino}phenyl)diazenyl]-4H-1,2,4-triazol-1-iums
- Salze des 1,4-Diallyl-5-[(4-{methyl[2-(4-aza-1-azoniabicyclo[2.2.2]octan-1-yl)ethyl]amino}phenyl)diazenyl]-4H-1,2,4-triazol-1-iums
- Salze des 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iums
- Salze des 1,4-Diallyl-5-[(4-{propyl[2-(4-aza-1-azoniabicyclo[2.2.2]octan-1-yl)ethyl]amino}phenyl)diazenyl]-4H-1,2,4-triazol-1-iums
- Salze des 1,4-Dibenzyl-5-[(4-{methyl[2-(4-aza-1-azoniabicyclo[2.2.2]octan-1-yl)ethyl]amino}phenyl)diazenyl]-4H-1,2,4-triazol-1-iums
- Salze des 1,4-Dibenzyl-5-[(4-{ethyl[2-(4-aza-1-azoniabicyclo[2.2.2]octan-1-yl)ethyl]amino}phenyl)diazenyl]-4H-1,2,4-triazol-1-iums
- Salze des 1,4-Dibenzyl-5-[(4-{propyl[2-(4-aza-1-azoniabicyclo[2.2.2]octan-1-yl)ethyl]amino}phenyl)diazenyl]-4H-1,2,4-triazol-1-iums
- Salze des 1,4-Dimethyl-5-[(2-methyl-4-{ethyl[2-(4-aza-1-azoniabicyclo[2.2.2]octan-1-yl)ethyl]amino}phenyl)diazenyl]-4H-1,2,4-triazol-1-iums
- Salze des 1,4-Dimethyl-5-[(2-methoxy-4-{ethyl[2-(4-aza-1-azoniabicyclo[2.2.2]octan-1-yl)ethyl]amino}phenyl)diazenyl]-4H-1,2,4-triazol-1-iums
- Salze des 1,4-Dimethyl-5-[(2-chlor-4-{ethyl[2-(4-aza-1-azoniabicyclo[2.2.2]octan-1-yl)ethyl]amino}phenyl)diazenyl]-4H-1,2,4-triazol-1-iums
- Salze des 1,4-Dimethyl-5-[(4-{methyl[3-(4-aza-1-azoniabicyclo[2.2.2]octan-1-yl)propyl]amino}phenyl)diazenyl]-4H-1,2,4-triazol-1-iums
- Salze des 1,4-Dimethyl-5-[(4-{ethyl[3-(4-aza-1-azoniabicyclo[2.2.2]octan-1-yl)propyl]amino}phenyl)diazenyl]-4H-1,2,4-triazol-1-iums
- Salze des 1,4-Dimethyl-5-[(4-{propyl[3-(4-aza-1-azoniabicyclo[2.2.2]octan-1-yl)propyl]amino}phenyl)diazenyl]-4H-1,2,4-triazol-1-iums
- Salze des 1,4-Diethyl-5-[(4-{methyl[3-(4-aza-1-azoniabicyclo[2.2.2]octan-1-yl)propyl]amino}phenyl)diazenyl]-4H-1,2,4-triazol-1-iums
- Salze des 1,4-Diethyl-5-[(4-{ethyl[3-(4-aza-1-azoniabicyclo[2.2.2]octan-1-yl)propyl]amino}phenyl)diazenyl]-4H-1,2,4-triazol-1-iums
- Salze des 1,4-Diethyl-5-[(4-{propyl[3-(4-aza-1-azoniabicyclo[2.2.2]octan-1-yl)propyl]amino}phenyl)diazenyl]-4H-1,2,4-triazol-1-iums
- Salze des 1,4-Diallyl-5-[(4-{methyl[3-(4-aza-1-azoniabicyclo[2.2.2]octan-1-yl)propyl]amino}phenyl)diazenyl]-4H-1,2,4-triazol-1-iums
- Salze des 1,4-Diallyl-5-[(4-{ethyl[3-(4-aza-1-azoniabicyclo[2.2.2]octan-1-yl)propyl]amino}phenyl)diazenyl]-4H-1,2,4-triazol-1-iums
- Salze des 1,4-Diallyl-5-[(4-{propyl[3-(4-aza-1-azoniabicyclo[2.2.2]octan-1-yl)propyl]amino}phenyl)diazenyl]-4H-1,2,4-triazol-1-iums
- Salze des 1,4-Dibenzyl-5-[(4-{methyl[3-(4-aza-1-azoniabicyclo[2.2.2]octan-1-yl)propyl]amino}phenyl)diazenyl]-4H-1,2,4-triazol-1-iums
- Salze des 1,4-Dibenzyl-5-[(4-{ethyl[3-(4-aza-1-azoniabicyclo[2.2.2]octan-1-yl)propyl]amino}phenyl)diazenyl]-4H-1,2,4-triazol-1-iums
- Salze des 1,4-Dibenzyl-5-[(4-{propyl[3-(4-aza-1-azoniabicyclo[2.2.2]octan-1-yl)propyl]amino}phenyl)diazenyl]-4H-1,2,4-triazol-1-iums

Als explizit besonders herausragend hervorzuheben sind innerhalb der ersten bevorzugten Ausführungsform wiederum die nachfolgenden Verbindungen:
- 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iumdichlorid
- 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iumdibromid
- 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iumbis-p-toluolsulfonat
- 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iumsulfat
- 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iumdichlorid
- 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iumdibromid
- 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iumbis-p-touolsulfonat
- 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iumsulfat
- 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iumdichlorid
- 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iumdibromid
- 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iumbis-p-toluolsulfonat
- 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iumsulfat

Im Rahmen einer zweiten bevorzugten Ausführungsform hat es sich zur Lösung der erfindungsgemäßen Aufgabenstellung ebenfalls als von besonderer Eignung erwiesen, wenn Y für =CH- steht.

Innerhalb dieser zweiten bevorzugten Ausführungsform enthalten besonders bevorzugte Mittel zum Färben und gegebenenfalls gleichzeitigen Aufhellen keratinischer Fasern mindestens eine Verbindung ausgewählt aus der Gruppe der
- Salze des 1,3-Dimethyl-2-[(4-{methyl[2-(4-aza-1-azoniabicyclo[2.2.2]octan-1-yl)ethyl]amino}phenyl)diazenyl]-1 H-imidazol-3-iums
- Salze des 1,3-Dimethyl-2-[(4-{ethyl[2-(4-aza-1-azoniabicyclo[2.2.2]octan-1-yl)ethyl]amino}phenyl)diazenyl]-1H-imidazol-3-iums
- Salze des 1,3-Dimethyl-2-[(4-{propyl[2-(4-aza-1-azoniabicyclo[2.2.2]octan-1-yl)ethyl]amino}phenyl)diazenyl]-1H-imidazol-3-iums
- Salze des 1,3-Diethyl-2-[(4-{methyl[2-(4-aza-1-azoniabicyclo[2.2.2]octan-1-yl)ethyl]amino}phenyl)diazenyl]-1H-imidazol-3-iums
- Salze des 1,3-Diethyl-2-[(4-{ethyl[2-(4-aza-1-azoniabicyclo[2.2.2]octan-1-yl)ethyl]amino}phenyl)diazenyl]-1H-imidazol-3-iums
- Salze des 1,3-Diethyl-2-[(4-{propyl[2-(4-aza-1-azoniabicyclo[2.2.2]octan-1-yl)ethyl]amino}phenyl)diazenyl]-1H-imidazol-3-iums
- Salze des 1,3-Diallyl-2-[(4-{methyl[2-(4-aza-1-azoniabicyclo[2.2.2]octan-1-yl)ethyl]amino}phenyl)diazenyl]-1H-imidazol-3-iums
- Salze des 1,3-Diallyl-2-[(4-{ethyl[2-(4-aza-1-azoniabicyclo[2.2.2]octan-1-yl)ethyl]amino}phenyl)diazenyl]-1H-imidazol-3-iums
- Salze des 1,3-Diallyl-2-[(4-{propyl[2-(4-aza-1-azoniabicyclo[2.2.2]octan-1-yl)ethyl]amino}phenyl)diazenyl]-1H-imidazol-3-iums
- Salze des 1,3-Dibenzyl-2-[(4-{methyl[2-(4-aza-1-azoniabicyclo[2.2.2]octan-1-yl)ethyl]amino}phenyl)diazenyl]-1H-imidazol-3-iums
- Salze des 1,3-Dibenzyl-2-[(4-{ethyl[2-(4-aza-1-azoniabicyclo[2.2.2]octan-1-yl)ethyl]amino}phenyl)diazenyl]-1H-imidazol-3-iums
- Salze des 1,3-Dibenzyl-2-[(4-{propyl[2-(4-aza-1-azoniabicyclo[2.2.2]octan-1-yl)ethyl]amino}phenyl)diazenyl]-1H-imidazol-3-iums
- Salze des 1,3-Dimethyl-2-[(2-methyl-4-{ethyl[2-(4-aza-1-azoniabicyclo[2.2.2]octan-1-yl)ethyl]amino}phenyl)diazenyl]-1H-imidazol-3-iums
- Salze des 1,3-Dimethyl-2-[(2-methoxy-4-{ethyl[2-(4-aza-1-azoniabicyclo[2.2.2]octan-1-yl)ethyl]amino}phenyl)diazenyl]-1H-imidazol-3-iums
- Salze des 1,3-Dimethyl-2-[(2-chlor-4-{ethyl[2-(4-aza-1-azoniabicyclo[2.2.2]octan-1-yl)ethyl]amino}phenyl)diazenyl]-1H-imidazol-3-iums
- Salze des 1,3-Dimethyl-2-[(4-{methyl[3-(4-aza-1-azoniabicyclo[2.2.2]octan-1-yl)propyl]amino}phenyl)diazenyl]-1H-imidazol-3-iums
- Salze des 1,3-Dimethyl-2-[(4-{ethyl[3-(4-aza-1-azoniabicyclo[2.2.2]octan-1-yl)propyl]amino}phenyl)diazenyl]-1H-imidazol-3-iums
- Salze des 1,3-Dimethyl-2-[(4-{propyl[3-(4-aza-1-azoniabicyclo[2.2.2]octan-1-yl)propyl]amino}phenyl)diazenyl]-1H-imidazol-3-iums
- Salze des 1,3-Diethyl-2-[(4-{methyl[3-(4-aza-1-azoniabicyclo[2.2.2]octan-1-yl)propyl]amino}phenyl)diazenyl]-1H-imidazol-3-iums
- Salze des 1,3-Diethyl-2-[(4-{ethyl[3-(4-aza-1-azoniabicyclo[2.2.2]octan-1-yl)propyl]amino}phenyl)diazenyl]-1H-imidazol-3-iums
- Salze des 1,3-Diethyl-2-[(4-{propyl[3-(4-aza-1-azoniabicyclo[2.2.2]octan-1-yl)propyl]amino}phenyl)diazenyl]-1H-imidazol-3-iums
- Salze des 1,3-Diallyl-2-[(4-{methyl[3-(4-aza-1-azoniabicyclo[2.2.2]octan-1-yl)propyl]amino}phenyl)diazenyl]-1H-imidazol-3-iums
- Salze des 1,3-Diallyl-2-[(4-{ethyl[3-(4-aza-1-azoniabicyclo[2.2.2]octan-1-yl)propyl]amino}phenyl)diazenyl]-1H-imidazol-3-iums
- Salze des 1,3-Diallyl-2-[(4-{propyl[3-(4-aza-1-azoniabicyclo[2.2.2]octan-1-yl)propyl]amino}phenyl)diazenyl]-1H-imidazol-3-iums
- Salze des 1,3-Dibenzyl-2-[(4-{methyl[3-(4-aza-1-azoniabicyclo[2.2.2]octan-1-yl)propyl]amino}phenyl)diazenyl]-1H-imidazol-3-iums
- Salze des 1,3-Dibenzyl-2-[(4-{ethyl[3-(4-aza-1-azoniabicyclo[2.2.2]octan-1-yl)propyl]amino}phenyl)diazenyl]-1H-imidazol-3-iums
- Salze des 1,3-Dibenzyl-2-[(4-{propyl[3-(4-aza-1-azoniabicyclo[2.2.2]octan-1-yl)propyl]amino}phenyl)diazenyl]-1H-imidazol-3-iums Innerhalb der Verbindungen der zweiten Ausführungsform nochmals als explizit besonders bevorzugt hervorzuheben sind die Verbindungen ausgewählt aus der Gruppe
- 1,3-Dimethyl-2-[(4-{ethyl[2-(4-aza-1-azoniabicyclo[2.2.2]octan-1-yl)ethyl]amino}phenyl)diazenyl]-1H-imidazol-3-iumdichlorid
- 1,3-Dimethyl-2-[(4-{ethyl[2-(4-aza-1-azoniabicyclo[2.2.2]octan-1-yl)ethyl]amino}phenyl)diazenyl]-1H-imidazol-3-iumdibromid
- 1,3-Dimethyl-2-[(4-{ethyl[2-(4-aza-1-azoniabicyclo[2.2.2]octan-1-yl)ethyl]amino}phenyl)diazenyl]-1H-imidazol-3-iumbis-p-toluolsulfonat
- 1,3-Dimethyl-2-[(4-{ethyl[2-(4-aza-1-azoniabicyclo[2.2.2]octan-1-yl)ethyl]amino}phenyl)diazenyl]-1H-imidazol-3-iumsulfat

Weitere besonders bevorzugte Salze sind weiter unten bei der Offenbarung der Verbindungen an sich explizit aufgeführt. Erfindungsgemäße Mittel, die die dort offenbarten Salze enthalten, sind ebenfalls besonders bevorzugt.

Die erfindungsgemäßen Mittel zum Färben und gegebenenfalls gleichzeitigen Aufhellen von Keratinfasern enthalten die Verbindung(en) der Formel (I) vorzugsweise in Mengen oberhalb von 1 ppm und unterhalb von 10 Gew.-%, jeweils bezogen auf das gesamte Mittel. Bevorzugte erfindungsgemäße Mittel zum Färben und gegebenenfalls gleichzeitigen Aufhellen keratinischer Fasern sind dadurch gekennzeichnet, daß sie die Verbindung(en) der Formel (I) in Mengen von 0,001 bis 5 Gew.-%, vorzugsweise von 0,0025 bis 2,5 Gew.-%, besonders bevorzugt von 0,005 bis 1,5 Gew.-% und insbesondere von 0,01 bis 1 Gew.-%, jeweils bezogen auf das gesamte Mittel, enthalten.

Die erfindungsgemäßen Mittel dienen der Farbveränderung keratinischer Fasern, insbesondere menschlicher Haare. Die Farbveränderung kann allein aufgrund der kationischen Verbindung(en) der Formel (I) erfolgen, die erfindungsgemäßen Mittel können aber auch zusätzlich weitere farbverändernde Substanzen enthalten, beispielsweise direktziehende Farbstoffe und/oder Oxidationsfärbemittel. Erfindungsgemäße Mittel zum Färben und gegebenenfalls gleichzeitigen Aufhellen keratinischer Fasern, die zusätzlich - bezogen auf ihr Gewicht - 0,001 bis 5 Gew.-% eines oder mehrerer Oxidationsfarbstoffvorprodukte und/oder direktziehender Farbstoffe enthalten, sind dabei bevorzugt.

Für permanente, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Diese weiteren farbgebenden Substanzen wurrden im Prioritätsdokument detailliert beschrieben. Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(a,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraamino-pyrimidin, 4-Hydroxy-2,5,6-triaminopyrimidin, 2-Hydroxy-4,5,6-triaminopyrimidin, sowie den physiologisch verträglichen Salzen dieser Verbindungen.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Entwicklerkomponente zusätzlich mindestens eine Kupplerkomponente zum Einsatz kommt.

Erfindungsgemäß besonders bevorzugte Kupplerkomponenten werden ausgewählt unter m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Für temporäre Färbungen werden üblicherweise Färbe- oder Tönungsmittel verwendet, die als färbende Komponente so genannte Direktzieher enthalten. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar ein sichtbarer homogener Farbverlust eintritt.

Weiterhin können die erfindungsgemäßen Mittel mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%.

Direktziehende Farbstoffe können in anionische, kationische und nichtionische direktziehende Farbstoffe unterteilt werden.

Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind.

Die erfindungsgemäßen Mittel enthalten mit besonderem Vorzug zusätzlich Wasserstoffperoxid. Hier sind erfindungsgemäße Mittel zum Färben und gegebenenfalls gleichzeitigen Aufhellen keratinischer Fasern besonders bevorzugt, die 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthalten.

Das Wasserstoffperoxid kann auch in Form von dessen Anlagerungsverbindungen an feste Träger eingesetzt werden, bevorzugt wird Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon n H₂O₂ (n ist eine positive ganze Zahl größer 0), Harnstoffperoxid und Melaminperoxid, eingesetzt. Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet.

Für eine Farbveränderung mittels Aufhellung bzw. Bleichung des Substrats, beispielsweise der Haare, wird bevorzugt in den erfindungsgemäßen kosmetischen Mitteln neben den Oxidationsmitteln zusätzlich mindestens ein Bleichverstärker eingesetzt.

Bleichverstärker werden bevorzugt zur Steigerung der Bleichwirkung des Oxidationsmittels, insbesondere des Wasserstoffperoxids, eingesetzt. Geeignete Bleichverstärker sind
(BV-i) Verbindungen, die unter Perhydrolysebedingungen aliphatische Peroxocarbonsäuren und/oder gegebenenfalls substituierte Perbenzoesäure ergeben, und/oder
(BV-ii) Carbonatsalze und/oder Hydrogencarbonatsalze, und/oder
(BV-iii) organische Carbonate, und/oder
(BV-iv) Carbonsäuren, und/oder
(BV-v) Peroxoverbindungen.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die sauerstoff- und/oder stichstoffgebundene Acylgruppen mit der genannten Anzahl an Kohlenstoffatomen und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Die Carbonat- bzw. Hydrogencarbonatsalze werden bevorzugt ausgewählt aus mindestens einer Verbindung der Gruppe bestehend aus Ammonium-, Alkali- (insbesondere Natrium- und Kalium-), sowie Erdalkali- (insbesondere Calcium-), -carbonatsalzen und -hydrogencarbonatsalzen. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Bevorzugt nutzbare organische Carbonate werden ausgewählt aus mindestens einer Verbindung der Gruppe der Kohlensäuremonoester und/oder aus mindestens einer Verbindung der Gruppe der Kohlensäuremonoamide.

Als bleichverstärkende Carbonsäure kann in den erfindungsgemäßen Zusammensetzungen bevorzugt mindestens eine Verbindung aus der Gruppe, bestehend aus Essigsäure, Milchsäure, Weinsäure, Zitronensäure, Salicylsäure und ortho-Phthalsäure, enthalten sein.

Bleichverstärker sind bevorzugt Peroxoverbindungen, insbesondere anorganische Peroxoverbindungen. Unter die erfindungsgemäß bleichverstärkenden Peroxoverbindungen fallen keine Anlagerungsprodukte von Wasserstoffperoxid an andere Komponenten und auch nicht Wasserstoffperoxid selbst. Die Auswahl der Peroxoverbindungen unterliegt darüber hinaus keinen Beschränkungen. Bevorzugte Peroxoverbindungen sind Peroxidisulfatsalze, Persulfatsalze, Peroxidiphosphatsalze (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die Peroxidisulfate, insbesondere Ammoniumperoxidisulfat, bevorzugt. Hier sind erfindungsgemäße Mittel zum Färben und gegebenenfalls gleichzeitigen Aufhellen keratinischer Fasern bevorzugt, die zusätzlich 0,01 bis 2 Gew.-% mindestens einer festen Peroxoverbindung, die ausgewählt ist aus Ammonium-, Alkalimetall- und Erdalkalimetallpersulfaten, - peroxomonosulfaten und -peroxidisulfaten enthalten, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei besonders bevorzugte Mittel mindestens zwei verschiedene Peroxidisulfate enthalten.

Weiterhin besonders bevorzugt sind Persulfate, insbesondere das als Carosche Salz bezeichnete Gemisch aus Kaliumperoxosulfat, Kaliumhydrogensulfat und Kaliumsulfat.

Die Bleichverstärker sind in den erfindungsgemäßen, kosmetischen Mitteln bevorzugt in Mengen von 5 bis 30 Gew.-%, insbesondere in Mengen von 8 bis 20 Gew.-%, jeweils bezogen auf das Gewicht des anwendungsbereiten Mittels, enthalten.

Weiterhin hat es sich als vorteilhaft erwiesen, wenn die erfindungsgemäßen Färbe- und/oder Aufhellmittel nichtionogene grenzflächenaktive Stoffe enthalten.

Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen. Besonders bervorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmitteln und anderen Begleitstoffen sein sollen.

Als weiteren Bestandteil können die erfindungsgemäßen Zusammensetzungen mindestens eine Ammoniumverbindung aus der Gruppe Ammoniumchlorid, Ammoniumcarbonat, Ammoniumbicarbonat, Ammoniumsulfat und/oder Ammoniumcarbamat in einer Menge von 0,5 bis 10, vorzugsweise 1 bis 5 Gew.-%, bezogen auf die Gesamtzusammensetzung des Mittels enthalten.

Ferner können die erfindungsgemäßen Färbe- und/oder Aufhellmittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Panthenol, Pantothensäure, Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch,

Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine, Fettalkohole und Fettsäureester,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die Auswahl dieser weiteren Stoffe wird der Fachmann gemäß der gewünschten Eigenschaften der Mittel treffen.

Die erfindungsgemäßen Mittel können die Inhaltsstoffe in einem geeigneten wäßrigen, alkoholischen oder wäßrig-alkoholischen Träger enthalten. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, eine pulverförmige oder auch Tabletten-förmige Formulierung bereitzustellen, was für Färbe- und/oder Aufhellmittel bevorzugt ist. Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Bevorzugte erfindungsgemäße Mittel sind dadurch gekennzeichnet, daß sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1 - 20 Gewichtsprozent, ganz besonders bevorzugt in einer Konzentration von 2 - 10 Gewichtsprozent, jeweils bezogen auf das Mittel, enthalten.

In weiter bevorzugten erfindungsgemäßen Mitteln ist das Lösungsmittel ausgewählt aus Ethanol, n-Propanol, Isoropanol, n-Butanol, Propylenglykol, n- Butylenglykol, Glycerin, Diethylenglykolmonoethylether, Diethylenglykolmono-n- butylether , Phenoxyethanol und Benzylalkohol sowie ihren Mischungen.

Der pH-Wert der erfindungsgemäßen Mittel kann durch geeignete Inhaltstoffe wie Acidifizierungsmittel oder Alkalisierungsmittel in einem weiten Bereich eingestellt werden.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprdukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, lodide, Chinone oder bestimmte Enzyme.

Geeignete Metallionen sind beispielsweise Zn²⁺, Cu²⁺, Fe²⁺ , Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Verbindungen der Formel (la) und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Für die Anwendung der erfindungsgemäßen Mittel auf dem Haar werden die Färbe- und/oder Aufhellmittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Färbe- und/oder Aufhellmittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

Zusätzlich können die erfindungsgemäßen Mittel weitere Inhaltsstoffe enthalten. Ein Einsatz bestimmter Metallionen oder -komplexe kann beispielsweise bevorzugt sein, um intensive Färbungen zu erhalten. Hier sind erfindungsgemäße Mittel bevorzugt, die zusätzlich Cu-, Fe-, Mn-, Ru-lonen oder Komplexe dieser Ionen enthalten.

Bevorzugte erfindungsgemäße Mittel zum Färben und gegebenenfalls gleichzeitigen Aufhellen keratinischer Fasern enthalten zusätzlich Cu-, Fe-, Mn-, Co-, Ce-, V-, Ru-lonen oder Komplexe dieser Ionen, wobei bevorzugte Mittel 0,0001 bis 2,5 Gew.-%, vorzugsweise 0,001 bis 1 Gew.-% mindestens einer Verbindung aus der Gruppe Kupferchlorid (CuCl₂), Kupfersulfat (CuSO₄), Eisen(II)sulfat, Mangan(II)sulfat, Mangan(II)chlorid, Kobalt(II)chlorid, Cersulfat, Cerchlorid, Vanadiumsulfat, Mangandioxid (MnO₂).

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Gebräuchliche und im Rahmen der vorliegenden Erfindung bevorzugte Chelatkomplexbilder sind beispielsweise Polyoxycarbonsäuren, Polyamine, Ethylendiamintetraessigsäure (EDTA), Nitrilotriessigsäure (NTA) und Hydroxyethandiphosphonsäuren bzw. deren Alkalisalze. Auch komplexbildende Polymere, also Polymere, die entweder in der Hauptkette selbst oder seitenständig zu dieser funktionelle Gruppen tragen, die als Liganden wirken können und mit geeigneten Metall-Atomen in der Regel unter Bildung von Chelat-Komplexen reagieren, sind erfindungsgemäß einsetzbar. Die Polymer-gebundenen Liganden der entstehenden MetallKomplexe können dabei aus nur einem Makromolekül stammen oder aber zu verschiedenen Polymerketten gehören. Letzteres führt zur Vernetzung des Materials, sofern die komplexbildenden Polymere nicht bereits zuvor über kovalente Bindungen vernetzt waren.

Komplexierende Gruppen (Liganden) üblicher komplexbildender Polymere sind Iminodiessigsäure-, Hydroxychinolin-, Thioharnstoff-, Guanidin-, Dithiocarbamat-, Hydroxamsäure-, Amidoxim-, Aminophosphorsäure-, (cycl.) Polyamino-, Mercapto-, 1,3-Dicarbonyl- und Kronenether-Reste mit z. T. sehr spezif. Aktivitäten gegenüber Ionen unterschiedlicher Metalle. Basispolymere vieler auch kommerziell bedeutender komplexbildender Polymere sind Polystyrol, Polyacrylate, Polyacrylnitrile, Polyvinylalkohole, Polyvinylpyridine und Polyethylenimine. Auch natürliche Polymere wie Cellulose, Stärke od. Chitin sind komplexbildende Polymere. Darüber hinaus können diese durch polymeranaloge Umwandlungen mit weiteren Ligand-Funktionalitäten versehen werden.

Besonders bevorzugt ist im Rahmen der vorliegenden Erfindung der Einsatz eines oder mehrerer Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine.

Erfindungsgemäß besonders bevorzugte Mittel enthalten einen oder mehrere Stoffe aus der Gruppe
(a) Nitrilotriessigsäure (NTA),
(b) Diethylenetriaminpentaesigsäure (DTPA),
(c) Ethylendiamindibernsteinsäure (EDDS),
(d) Ethylenediamindiglutarsäure (EDGA),
(e) 2- Hydroxypropylendiamindibernsteinsäure (HPDS),
(f) Glycinamid-N,N'- dibernsteinsäure (GADS),
(g) Ethylendiamin-N-N'-diglutarsäure (EDDG),
(h) 2-Hydroxypropylendiamin-N-N'-dibernsteinsäure (HPDDS),
(i) Ethylendiamintetraessigsäure (EDTA),
(j) Ethylendicysteinsäure (EDC),
(k) Diaminoalkyldi(sulfobernsteinsäure) (DDS),
(l) Ethylendiamine-N-N'-bis(ortho-hydroxyphenylesigsäure (EDDHA),
(m) N-2-hydroxyethyl-N,N-diessigsäure,
(n) Glyceryliminodiessigsäure,
(o) Iminodiessigsäure-N-2-hydroxypropylsulfonsäure,
(p) Asparaginsäure-N-carboxymethyl-N-2,5-hydroxypropyl-3-sulfonsäure,
(q) 8-Alanin-N,N'-diessigsäure,
(r) Asparaginsäure-N,N'-diessigsäure,
(s) Asparaginsäure-N-monoessigsäure,
(t) Dipicolinsäure,
(u) sowie deren Salze und/oder Derivate

Aus den vorstehend genannten Stoffgruppen sind einige Vertreter im Rahmen der vorliegenden Erfindung besonders bevorzugt. Erfindungsgemäß besonders bevorzugte Mittel zum Färben und gegebenenfalls gleichzeitigen Aufhellen keratinischer Fasern enthalten zusätzlich einen oder mehrere Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine,
wobei bevorzugte Mittel Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthalten.

Bevorzugte erfindungsgemäße Mittel werden wasserarm bzw. wasserfrei formuliert. Erfindungsgemäß besonders bevorzugte Mittel sind dadurch gekennzeichnet, daß sie weniger als 5 Gew.-%, vorzugsweise weniger als 2 Gew.-%, besonders bevorzugt weniger als 1 Gew.-% und insbesondere weniger als 0,5 Gew.-% Wasser enthalten, wobei bevorzugte Mittel wasserfrei sind. Der Wassergehalt der Mittel läßt sich beispielsweise mittels Titration nach Karl Fischer bestimmen.

Ein zweiter Gegenstand der vorliegenden Erfindung ist ein Verfahren zum Färben und gegebenenfalls gleichzeitigen Aufhellen von Keratinfasern, insbesondere menschlichen Haaren, bei dem
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein erfindungsgemäßes Mittel ist.

Die erfindungsgemäßen Mittel können demnach als Einkomponentenmittel (Färbe- und Aufhellmittel M2 bzw. Nachbehandlungsmittel M4), als Zweikomponenten Mittel (M2 + M3) oder als Dreikomponentenmittel (M2 + M3 + M4) formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Färbe- und Aufhellungsverfahren, bei dem die Aufhellcreme und das Oxidationsmittel zunächst getrennt vorliegen, ist dabei bevorzugt. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem erfindungsgemäßen Mittel zu einer homogenen Zusammensetzung vermischt, und diese auf das Haar aufgebracht wird.

In bevorzugten erfindungsgemäßen Verfahren dieser Art enthält die Zusammensetzung auf wäßriger Grundlage bezogen auf ihr Gewicht 1 bis 20 Gew.-%, vorzugsweise 2 bis 10 Gew.-% und insbesondere 3 bis 6 Gew.-% Wasserstoffperoxid, berechnet als 100%iges H₂O₂.

Weiter bevorzugte erfindungsgemäße Verfahren dieser Art sind dadurch gekennzeichnet, daß die Zusammensetzung auf wäßriger Grundlage, enthaltend Wasserstoffperoxid, mit einem erfindungsgemäßen Mittel im Gewichtsverhältnis 1:5 bis 10:1, vorzugsweise 1:2 bis 5:1 und insbesondere 1:2 bis 2:1 zu einer homogenen Zusammensetzung vermischt, und diese auf das Haar aufgebracht wird.

Alternativ kann - wie vorstehend erwähnt - auch ein Dreikomponentensystem zur Anwendung gelangen. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem weiteren Mittel enthaltend vorzugsweise mindestens einen Alkalitätsgeber und/oder direktziehenden Haarfarbstoff und/oder mindestens ein Oxidationsfarbstoffvorprodukt, und einem erfindungsgemäßen Mittel zu einer homogenen Zusammensetzung vermischt, und diese auf das Haar aufgebracht wird.

Bezüglich weiterer bevorzugter Ausführungsformen der erfindungsgemäßen Verfahren gilt mutatis mutandis das zu den erfindungsgemäßen Mitteln Gesagte.

Besonders bevorzugt ist es, die Ausfärbung durch physikalische Maßnahmen zu unterstützen. Erfindungsgemäße Verfahren, bei denen die Anwendung durch Einwirkung von Wärme und/oder UV-Strahlung während der Einwirkzeit unterstützt wird, sind bevorzugte Ausführungsformen der vorliegenden Erfindung.

Die Verbindungen der allgemeinen Formel (I) eignen sich sehr gut als Direktzieher für die Haarfärbung. In Ausfärbungen werden extrem intensive Farbnuancen mit sehr guten Echtheitseigenschaften, insbesondere im Gelb-, Orange- und Rotbereich erhalten. Die Direktzieher liefern ebenfalls bei gleichzeitiger Anwendung von Oxidationsmitteln wie Wasserstoffperoxid oder einem Gemisch aus Wasserstoffperoxid und Peroxidisulfaten leuchtende Nuancen ohne Abschwächung von Farbintensität und Farbbrillanz. Auf diese Weise wird das gleichzeitige Aufhellen und Färben von Haaren möglich, wodurch auch auf dunklem Haar eine leuchtende Farbgebung erzielt werden kann.

Ein weiterer Gegenstand der vorliegenden Erfindung ist daher die Verwendung von dikationischen Azofarbstoffen der Formel (I): 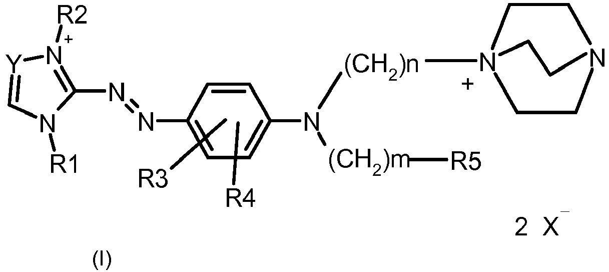 in der
- Y: steht entweder für =CH- oder ein Stickstoffatom,
- R¹, R²: stehen unabhängig voneinander für eine C₁-C₆-Alkylgruppe, eine C₂-C₆-Alkenylgruppe, eine Hydroxy-C₁-C₆-alkylgruppe, eine Polyhydroxy-C₂-C₆-alkylgruppe, eine C₁-C₆-Alkoxy-C₂-C₆-alkylgruppe, eine Cyano-C₁-C₆-alkylgruppe, eine Arylgruppe oder eine Aryl-C₁-C₆-alkylgruppe,
- R³, R⁴: stehen unabhängig voneinander für ein Wasserstoffatom, eine C₁-C₆-Alkylgruppe, ein Halogenatom (Fluor, Chlor, Brom oder Iod), eine C₁-C₆-Alkoxygruppe, eine Aminogruppe, eine C₁-C₆-Alkylaminogruppe, eine Di-C₁-C₆-alkylaminogruppe, eine Hydroxygruppe oder aber R³ und R⁴ bilden zusammen einen 5-,6- oder 7-gliedrigen, gesättigten oder unsgesättigten Ring, der gegebenenfalls weitere Heteroatome enthalten oder weitere Substituenten tragen kann kann,
- n,m: stehen unabhängig voneinander für eine ganze Zahl von 1 bis 6,
- R⁵: steht für ein Wasserstoffatom, eine C₁-C₆-Alkoxygruppe, ein Halogenatom (Fluor, Chlor, Brom oder Iod), eine Hydroxygruppe, eine C₁-C₆-Alkoxy-C₂-C₆-alkyloxygruppe, eine Nitrilgruppe, eine Arylgruppe oder eine Heteroarylgruppe,
- X-: steht für ein einwertiges Anion, vorzugsweise für Halogenid, Hydrogensulfat, ½ Sulfat, Benzolsulfonat, p-Toluolsulfonat, Acetat, Citrat, Lactat, Tetrafluoroborat, Trifluormethansulfonat, Hexafluorophosphat
in Mitteln zum Färben und gegebenenfalls gleichzeitigen Aufhellen keratinischer Fasern. Besonders bevorzugt dient der Einsatz der Verbindungen der Erzielung bestimmter vorteilhafter Effekte. Erfindungsgemäß bevorzugt sind Verwendungen zur
- Steigerung der Echtheitseigenschaften der Färbungen und/oder
- Steigerung der Farbbrillanz und/oder
- Steigerung der Hautverträglichkeit von Färbe bzw. Aufhellmitteln.

Bezüglich weiterer bevorzugter erfindungsgemäßer Verwendungen gilt mutatis mutandis das zu den bevorzugten Mitteln Ausgeführte.

Die in den erfindungsgemäßen Mitteln enthaltenen kationischen Verbindungen sind bislang nicht bekannt. Ein weiterer Gegenstand der vorliegenden Erfindung sind demnach dikationische 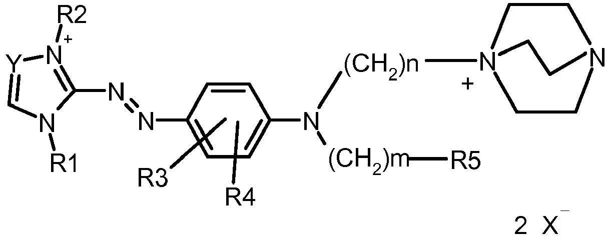 Azofarbstoffe der Formel (I): in der
- Y: steht entweder für =CH- oder ein Stickstoffatom,
- R¹, R²: stehen unabhängig voneinander für eine C₁-C₆-Alkylgruppe, eine C₂-C₆-Alkenylgruppe, eine Hydroxy-C₁-C₆-alkylgruppe, eine Polyhydroxy-C₂-C₆-alkylgruppe, eine C₁-C₆-Alkoxy-C₂-C₆-alkylgruppe, eine Cyano-C₁-C₆-alkylgruppe, eine Arylgruppe oder eine Aryl-C₁-C₆-alkylgruppe,
- R³, R⁴: stehen unabhängig voneinander für ein Wasserstoffatom, eine C₁-C₆-Alkylgruppe, ein Halogenatom (Fluor, Chlor, Brom oder Iod), eine C₁-C₆-Alkoxygruppe, eine Aminogruppe, eine C₁-C₆-Alkylaminogruppe, eine Di-C₁-C₆-alkylaminogruppe, eine Hydroxygruppe oder aber R³ und R⁴ bilden zusammen einen 5-,6- oder 7-gliedrigen, gesättigten oder unsgesättigten Ring, der gegebenenfalls weitere Heteroatome enthalten oder weitere Substituenten tragen kann kann,
- n,m: stehen unabhängig voneinander für eine ganze Zahl von 1 bis 6,
- R⁵: steht für ein Wasserstoffatom, eine C₁-C₆-Alkoxygruppe, ein Halogenatom (Fluor, Chlor, Brom oder Iod), eine Hydroxygruppe, eine C₁-C₆-Alkoxy-C₂-C₆-alkyloxygruppe, eine Nitrilgruppe, eine Arylgruppe oder eine Heteroarylgruppe,
- X-: steht für ein einwertiges Anion, vorzugsweise für Halogenid, Hydrogensulfat, ½ Sulfat, Benzolsulfonat, p-Toluolsulfonat, Acetat, Citrat, Lactat, Tetrafluoroborat, Trifluormethansulfonat, Hexafluorophosphat

Ganz besonders bevorzugte erfindungsgemäße kationische Derivate sind ausgewählt aus
- Salzen des 1,4-Dimethyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-methyl-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-methoxy-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-chlor-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Dimethyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Dimethyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,3-Dimethyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-methyl-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-methoxy-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-chlor-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Dimethyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Dimethyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{propyl[3-(4-aza-1-azoniabicyclo[2.2.2]octan-1-yl)propyl]amino}phenyl)diazenyl]-1H-imidazol-3-iums

### Beispiele

Die Synthese von 1,4-Dimethyl-5-[(4-{ethyl[2-(4-aza-1-azoniabicyclo-[2.2.2]octan-1-yl)ethyl]amino}phenyl)diazenyl]-4H-1,2,4-triazol-1-iumbis-p-toluolsulfonat (DZ1) und von 1-[2-(N-Ethyl-N-phenylamino)ethyl]-4-aza-1-azoniabicyclo[2.2.2]octan, Chlorid wurde im Prioritätsdokument detailliert beschrieben.

Es wurde folgende Färbecremes hergestellt:

### 1.1.1 Nichtionische Färbecreme

| | |
|---|---|
| Hydrenol^{®} D | 6,0 g |
| Lorol^{®} (techn.) | 6,0 g |
| Eumulgin^{®} RH 40 | 1,0 g |
| Eumulgin^{®} B1 | 3,0 g |
| Eumulgin^{®} B2 | 3,0 g |
| PHB-Methylester | 0,3 g |
| PHB-Propylester | 0,2 g |
| Phenoxyethanol | 1,0 g |
| Polydiol^{®} 400 | 5,0 g |
| Direktzieher | 1,0 g |
| Ammoniumsulfat | 1,0 g (in 30,0 g Wasser) |
| Natrosol^{®} 250 HR | 1,0 g (in 15,0 g Wasser) |
| NaOH 0,1% | pH-Wert Einstellung |
| Wasser | ad 100 g |

Die ersten neun Komponenten wurden zusammen bei 80 °C aufgeschmolzen, danach wurde der Farbstoff zugefügt. Diese Mischung wurde mit einer Lösung des Ammoniumsulfats in 30 g Wasser emulgiert. Anschließend wurde eine Quellung von 1,0 g Natrosol^{®} 250 HR in 15,0 g Wasser hinzugegeben. Der in der Tabelle 2 angegebene pH-Wert wurde mit 0,1% Natronlauge eingestellt, anschließend wurde mit Wasser auf 100 g aufgefüllt.

### 1.1.2 Kationische Färbecreme

| | |
|---|---|
| Stenol^{®} 16/18 | 4,0 g |
| Eumulgin^{®} B1 | 1,0 g |
| Dehyquart^{®} A-CA | 2,0 g |
| Ammoniumsulfat | 1,0 g (in 30,0 g Wasser) |
| Direktzieher | 1,0 g |
| Wasser | at 100 g |

Das Stenol^{®} 16/18 wurde zusammen mit Eumulgin^{®} B1 und Dehyquart^{®} A-CA aufgeschmolzen, danach wurde die Schmelze mit heißem Wasser emulgiert. Dann wurden der Farbstoff sowie die wässrige Ammoniumsulfatlösung hinzugegeben. Der pH-Wert wurde mit Ammoniak bwz. Zitronensäure auf den in der Tabelle angegebenen Wert eingestellt, anschließend wurde mit Wasser wurde auf 100 g aufgefüllt.

### 1.1.3 Anionische Färbecreme

| | |
|---|---|
| Hydrenol^{®} D | 1,0 g |
| Lorol^{®} techn. | 1,0 g |
| Akypo Soft^{®} RLM 45N | 1,1 g |
| PHB-Propylester | 0,1 g |
| PHB-Methylester | 0,1 g |
| Ammoniumsulfat | 1,0 g (in 30,0 g Wasser) |
| Direktzieher DZ | 1,0 g |
| Wasser | at 100g |

Die ersten fünf Komponenten wurden zusammen aufgeschmolzen. Diese Schmelze wurde mit heißem Wasser emulgiert, anschließend wurde der in Wasser vorgelöste bzw. vordispergierte Farbstoff hinzugegeben sowie die Ammoniumsulfatlösung hinzugefügt. Der in der Tabelle angegebene pH-Wert wurde mit Ammoniak bzw. Zitronensäuer eingestellt, danach wurde mit Wasser auf 100 g aufgefüllt.

### 1.2 Verzeichnis der eingesetzten Rohstoffe

| | |
|---|---|
| Akypo RLM 45 NV^{®} | Laurylalkohol-4.5-EO-Essigsäure-Natrium-Salz (mind. 22% Aktivsubstanzgehalt; INCI-Bezeichnung: Sodium Laureth-5 Carboxylate) (Chem-Y) |
| Dehyquart^{®} A-CA | Trimethylhexadecylammoniumchlorid (ca. 24 -26 % Aktivsubstanz; INCI-Bezeichnung: Aqua (Water), Cetrimonium Chloride) (Cognis) |
| Eumulgin^{®} B 1 | Cetylstearylalkohol mit ca. 12-EO-Einheiten (INCI-Bezeichnung: Ceteareth-12) (Cognis) |
| Eumulgin^{®} B2 | Cetylstearylalkohol mit ca. 20 EO-Einheiten (INCI-Bezeichnung: Ceteareth-20) (Cognis) |
| Eumulgin^{®} RH 40 | gehärtetes Rizinusöl mit ca. 40-EO-Einheiten (INCI-Bezeichnung: PEG-40 Hydrogenated Castor Oil) (Cognis) |
| Hydrenol^{®} D | C₁₆₋₁₈-Fettalkohol (INCI-Bezeichnung: Cetearyl alcohol) (Cognis) |
| Lorol (techn.)^{®} | C₁₂₋₁₈-Fettalkohol (INCI-Bezeichnung: Coconut Alcohol) (Cognis) |
| Natrosol^{®} 250 HR | Hydroxyethylcellulose (INCI-Bezeichnung: Hydroxyethylcellulose) (Hercules) |
| Polydiol^{®} 400 | Polyethylenglykol (INCI-Bezeichung: PEG-8) (Cognis) |
| Stenol^{®} 1618 | C₁₆₋₁₈-Fettalkohol (INCI-Bezeichnung: Cetearyl Alcohol) (Cognis) |

### 1.3 Ausfärbungen

Jeweils 1,8 g der Färbecreme wurden auf eine ca. 6 cm lange Strähne Menschenhaares (Kerling Euronaturhaar, blond) aufgetragen und dort 30 Minuten bei 30 °C belassen. Nach Beendigung der Einwirkzeit wurde das Haar ausgespült, mit einem üblichen Haarwaschmittel gewaschen und anschließend getrocknet. Die Haarsträhnen wurden in den in den nachfolgenden Tabellen angegebenen Nuancen gefärbt.

### DZ1: 1,4-Dimethyl-5-[(4-{ethyl[2-(4-aza-1-azoniabicyclo-[2.2.2]octan-1-yl)ethyl]-amino}phenyl)diazenyl]-4H-1,2,4-triazol-1-iumbis-p-toluolsulfonat

| **Farbstoff** | **Färbecreme** | **pH-Wert** | **Farbnuance (Intensität)** |
|---|---|---|---|
| DZ 1 | 1 | 7,0 | erdbeerrot (+++) |
| DZ 1 | 1 | 9,0 | blutrot (+++) |
| DZ 1 | 2 | 7,0 | erdbeerrot (+++) |
| DZ 1 | 2 | 9,0 | blutrot (+++) |
| DZ 1 | 3 | 7,0 | erdbeerrot (+++) |
| DZ 1 | 3 | 9,0 | blutrot (+++) |

| | | | |
|---|---|---|---|
| Intensität: + = niedrig ++ = mittel +++ = hoch | | | |

### 2.4. Nachweis der Oxidationsstabilität

Aus den aufgelisteten Bestandteilen wurde eine Blondiercreme hergestellt:

| Rohstoff | Gew.-% |
|---|---|
| | |
| Hydrenol D | 12,00 |
| Lorol. techn. | 2,40 |
| Texapon NSO | 26,50 |
| Stabylen 30 | 0,10 |
| Cetiol OE | 2,40 |
| Turpinal SL | 0,20 |
| Direktzieher DZ | 2,00 |
| Natriumsilikat 40/42 | 0,50 |
| Ammoniumsulfat | 1,00 |
| Ammoniak, 25% | 7,60 |
| Wasser | ad 100 |

| | |
|---|---|
| Hydrenol^{®} D | INCI-Bezeichnung: Cetearyl alcohol (Cognis) |
| Lorol^{®} tech. | INCI-Bezeichnung: Coconut alcohol (Cognis) |
| Texapon^{®} NSO ca. 27,5% Aktivsubstanz; INCI-Bezeichnung: Sodium Laureth Sulfate (Cognis) | |
| Stabylen^{®} 30 | INCI-Bezeichnung: Acrylates/Vinylisodecanoate Crosspolymer (3V Sigma) |
| Cetiol OE | INCI-Bezeichnung: Dicaprylether (Cognis) |
| Turpinal^{®} SL | ca. 58 - 61% Aktivsubstanzgehalt; INCI-Bezeichnung: Etidronic Acid, Aqua (Solutia) |

Hydrenol D und Lorol wurden zusammen bei 80 °C aufgeschmolzen, dann wurden Texapon NSO, Stabylen 30, Cetiol OE und Turpinal SL der Reihe nach unter Rühren eingearbeitet. Natriumsilikat 40/42 und Ammoniumsulfat wurden jeweils in einer kleinen Menge Wasser vorgelöst und ebenfalls unter Rühren hinzugefügt. Dann wurde der erfindungsgemäße Direktzieher in einer kleinen Menge Wasser vorgelöst und ebenfalls unter Rühren zu der Formulierung gegeben.Bei einer Temperatur von ca. 40 °C wurde schließlich der Ammoniak hinzugefügt. Unter Rühren wurde mit Wasser auf 100 % aufgefüllt und die Formulierung kalt gerührt.

Diese Blondiercremes wurden jeweils im Verhältnis 1:1 mit einer wie folgt zusammengesetzten Entwicklerdispersion vermischt:

| Rohstoff | Gew.-% |
|---|---|
| Ammoniak, 25 % | 0,62 |
| Dipicolinsäure | 0,10 |
| Dinatriumpyrophosphat | 0,03 |
| Turpinal SL | 1,50 |
| Texapon NSO | 2,00 |
| Dow Corning DB 110 A (nicht ionische Silikonemulsion) | 0,07 |
| Aculyn 33A (Acrylpolymer) | 12,00 |
| Wasserstoffperoxid 50 % | 22,40 |
| Wasser | ad 100 |

Aculyn^{®} 33A ca. 28% Festkörper in Wasser; INCI-Bezeichnung: Acrylates Copolymer

100 g der Blondiercreme wurden mit 100 g der Entwicklerdispersion und 20 g einer Mischung aus Ammoniumperoxodisulfat, Natriumperoxodisulfat und Kaliumperoxodisulfat (je ein Gewichtsteil) vermengt. Der pH-Wert der fertigen Formulierung lag zwischen 9 und 10. Jeweils 1,8 g der auf diese Weise hergestellten fertigen Anwendungsmischung wurden auf eine ca. 6 cm lange Strähne Menschenhaares (Kerling Euronaturhaar, blond) aufgetragen und dort für 30 Minuten bei 30 °C belassen. Nach Beendigung der Einwirkzeit wurde das Haar ausgespült, mit einem üblichen Haarwaschmittel gewaschen und anschließend getrocknet. Die Haarsträhnen wurden in den nachfolgend angegebenen Nuancen gefärbt.
DZ1: 1,4-Dimethyl-5-[(4-{ethyl[2-(4-aza-1-azoniabicyclo-[2.2.2]octan-1-yl)ethyl]-amino}phenyl)diazenyl]-4H-1,2,4-triazol-1-iumbis-p-toluolsulfonat (erfindungsgemäß)
DZ2: 1-[2-({4-[2-(1,2,4-4H-Triazol-3-yl)diazenyl]phenyl}ethylamino)ethyl]-4-aza-1-azoniabicyclo[2.2.2]octan, Chlorid
(nicht erfindungsgemäß, Produkt aus Synthesebeispiel 1.3.)

| Farbstoff | Farbnuance (Intensität) ohne Wasserstoffperoxid/ Persulfat | Farbnuance (Intensität) mit Wasserstoffperoxid / Persulfat |
|---|---|---|
| DZ 1 (erfindungsgmemäß) | blutrot (+++) | blutrot (+++) |
| DZ 2 (Vergleich, nicht erfindungsgemäß) | indischgelb (+++) | schwach beige gelb |

## Patentansprüche

1. Mittel zum Färben und gegebenenfalls gleichzeitigen Aufhellen von Keratinfasern,
insbesondere menschlichen Haaren, enthaltend mindestens einen dikationischen 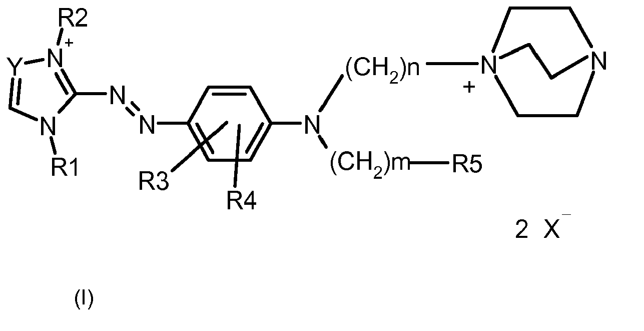 Azofarbstoff der Formel (I):
in der
Y steht entweder für =CH- oder ein Stickstoffatom,
R¹, R² stehen unabhängig voneinander für eine C₁-C₆-Alkylgruppe, eine C₂-C₆-Alkenylgruppe, eine Hydroxy-C₁-C₆-alkylgruppe, eine Polyhydroxy-C₂-C₆-alkylgruppe, eine C₁-C₆-Alkoxy-C₂-C₆-alkylgruppe, eine Cyano-C₁-C₆-alkylgruppe, eine Arylgruppe oder eine Aryl-C₁-C₆-alkylgruppe,
R³, R⁴ stehen unabhängig voneinander für ein Wasserstoffatom, eine C₁-C₆-Alkylgruppe, ein Halogenatom (Fluor, Chlor, Brom oder lod), eine C₁-C₆-Alkoxygruppe, eine Aminogruppe, eine C₁-C₆-Alkylaminogruppe, eine Di-C₁-C₆-alkylaminogruppe, eine Hydroxygruppe oder aber R³ und R⁴ bilden zusammen einen 5-,6- oder 7-gliedrigen, gesättigten oder unsgesättigten Ring, der gegebenenfalls weitere Heteroatome enthalten oder weitere Substituenten tragen kann kann,
n,m stehen unabhängig voneinander für eine ganze Zahl von 1 bis 6,
R⁵ steht für ein Wasserstoffatom, eine C₁-C₆-Alkoxygruppe, ein Halogenatom (Fluor, Chlor, Brom oder Iod), eine Hydroxygruppe, eine C₁-C₆-Alkoxy-C₂-C₆-alkyloxygruppe, eine Nitrilgruppe, eine Arylgruppe oder eine Heteroarylgruppe,
X- steht für ein einwertiges Anion, vorzugsweise für Halogenid, Hydrogensulfat, ½ Sulfat, Benzolsulfonat, p-Toluolsulfonat, Acetat, Citrat, Lactat, Tetrafluoroborat, Trifluormethansulfonat, Hexafluorophosphat.

2. Mittel zum Färben und gegebenenfalls gleichzeitigen Aufhellen keratinischer Fasern nach Anspruch 1, **dadurch gekennzeichnet, dass** es Verbindungen der Formel (I) enthält, in der die Reste R¹ und R² für eine C₁-C₆-Alkylgruppe (insbesondere eine Methyl- oder Ethylgruppe), eine C₂-C₆-Alkenylgruppe (insbesondere eine Allylgruppe) oder eine Aryl-C₁-C₆-alkylgruppe (insbesondere eine Benzylgruppe) stehen.

3. Mittel zum Färben und gegebenenfalls gleichzeitigen Aufhellen keratinischer Fasern nach einem der Ansprüche 1 bis 2, **dadurch gekennzeichnet, dass** es mindestens eine kationische Verbindung aus der Gruppe der dikationischen Azofarbstoffe, die gebildet wird aus
- Salzen des 1,4-Dimethyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-methyl-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-methoxy-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-chlor-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Dimethyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Dimethyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{propyl[3-(4-aza-1-azoniabicyclo[2.2.2]octan-1-yl)propyl]amino}phenyl)diazenyl]-4H-1,2,4-triazol-1-iums
enthält.

4. Mittel zum Färben und gegebenenfalls gleichzeitigen Aufhellen keratinischer Fasern nach einem der Ansprüche 1 bis 3, **dadurch gekennzeichnet, dass** es mindestens eine kationische Verbindung aus der Gruppe der dikationischen Azofarbstoffe, die gebildet wird aus
- Salzen des 1,3-Dimethyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-methyl-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-methoxy-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-chlor-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Dimethyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Dimethyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{propyl[3-(4-aza-1-azoniabicyclo[2.2.2]octan-1-yl)propyl]amino}phenyl)diazenyl]-1H-imidazol-3-iums
enthält.

5. Mittel zum Färben und gegebenenfalls gleichzeitigen Aufhellen keratinischer Fasern nach einem der Ansprüche 1 bis 4, **dadurch gekennzeichnet, dass** es die Verbindung(en) der Formel (I) in Mengen von 0,001 bis 5 Gew.-%, vorzugsweise von 0,0025 bis 2,5 Gew.-%, besonders bevorzugt von 0,005 bis 1,5 Gew.-% und insbesondere von 0,01 bis 1 Gew.-%, jeweils bezogen auf das gesamte Mittel, enthält.

6. Mittel zum Färben und gegebenenfalls gleichzeitigen Aufhellen keratinischer Fasern nach einem der Ansprüche 1 bis 5, **dadurch gekennzeichnet, dass** es zusätzlich - bezogen auf sein Gewicht - 0,001 bis 5 Gew.-% eines oder mehrerer Oxidationsfarbstoffvorprodukte und/oder direktziehender Farbstoffe enthält.

7. Mittel zum Färben und gegebenenfalls gleichzeitigen Aufhellen keratinischer Fasern nach einem der Ansprüche 1 bis 6, **dadurch gekennzeichnet, dass** es 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthält.

8. Verfahren zum Färben und gegebenenfalls gleichzeitigen Aufhellen von Keratinfasern, insbesondere menschlichen Haaren, **dadurch gekennzeichnet, dass**,
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein Mittel nach einem der Ansprüche 1 bis 6 ist.

9. Verwendung von dikationischen Azofarbstoffen der Formel (I): 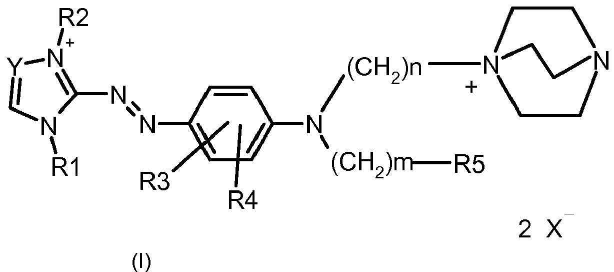 in der
Y steht entweder für =CH- oder ein Stickstoffatom,
R¹, R² stehen unabhängig voneinander für eine C₁-C₆-Alkylgruppe, eine C₂-C₆-Alkenylgruppe, eine Hydroxy-C₁-C₆-alkylgruppe, eine Polyhydroxy-C₂-C₆-alkylgruppe, eine C₁-C₆-Alkoxy-C₂-C₆-alkylgruppe, eine Cyano-C₁-C₆-alkylgruppe, eine Arylgruppe oder eine Aryl-C₁-C₆-alkylgruppe,
R³, R⁴ stehen unabhängig voneinander für ein Wasserstoffatom, eine C₁-C₆-Alkylgruppe, ein Halogenatom (Fluor, Chlor, Brom oder Iod), eine C₁-C₆-Alkoxygruppe, eine Aminogruppe, eine C₁-C₆-Alkylaminogruppe, eine Di-C₁-C₆-alkylaminogruppe, eine Hydroxygruppe oder aber R³ und R⁴ bilden zusammen einen 5-,6- oder 7-gliedrigen, gesättigten oder unsgesättigten Ring, der gegebenenfalls weitere Heteroatome enthalten oder weitere Substituenten tragen kann kann,
n,m stehen unabhängig voneinander für eine ganze Zahl von 1 bis 6,
R⁵ steht für ein Wasserstoffatom, eine C₁-C₆-Alkoxygruppe, ein Halogenatom (Fluor, Chlor, Brom oder Iod), eine Hydroxygruppe, eine C₁-C₆-Alkoxy-C₂-C₆-alkyloxygruppe, eine Nitrilgruppe, eine Arylgruppe oder eine Heteroarylgruppe,
X- steht für ein einwertiges Anion, vorzugsweise für Halogenid, Hydrogensulfat, ½ Sulfat, Benzolsulfonat, p-Toluolsulfonat, Acetat, Citrat, Lactat, Tetrafluoroborat, Trifluormethansulfonat, Hexafluorophosphat
in Mitteln zum Färben und gegebenenfalls gleichzeitigen Aufhellen keratinischer Fasern.

10. Dikationische Azofarbstoffe der Formel (I): 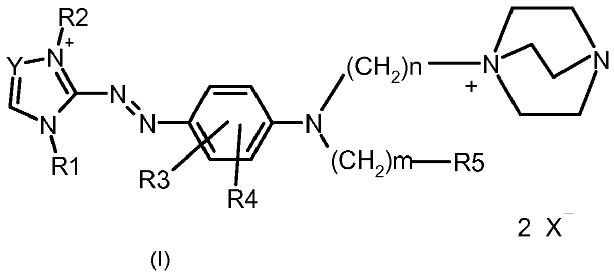 in der
Y steht entweder für =CH- oder ein Stickstoffatom,
R¹, R² stehen unabhängig voneinander für eine C₁-C₆-Alkylgruppe, eine C₂-C₆-Alkenylgruppe, eine Hydroxy-C₁-C₆-alkylgruppe, eine Polyhydroxy-C₂-C₆-alkylgruppe, eine C₁-C₆-Alkoxy-C₂C₆-alkylgruppe, eine Cyano-C₁-C₆-alkylgruppe, eine Arylgruppe oder eine Aryl-C₁-C₆-alkylgruppe,
R³, R⁴ stehen unabhängig voneinander für ein Wasserstoffatom, eine C₁-C₆-Alkylgruppe, ein Halogenatom (Fluor, Chlor, Brom oder Iod), eine C₁-C₆-Alkoxygruppe, eine Aminogruppe, eine C₁-C₆-Alkylaminogruppe, eine Di-C₁-C₆-alkylaminogruppe, eine Hydroxygruppe oder aber R³ und R⁴ bilden zusammen einen 5-,6- oder 7-gliedrigen, gesättigten oder unsgesättigten Ring, der gegebenenfalls weitere Heteroatome enthalten oder weitere Substituenten tragen kann kann,
n,m stehen unabhängig voneinander für eine ganze Zahl von 1 bis 6,
R⁵ steht für ein Wasserstoffatom, eine C₁-C₆-Alkoxygruppe, ein Halogenatom (Fluor, Chlor, Brom oder lod), eine Hydroxygruppe, eine C₁-C₆-Alkoxy-C₂-C₆-alkyloxygruppe, eine Nitrilgruppe, eine Arylgruppe oder eine Heteroarylgruppe,
X- steht für ein einwertiges Anion, vorzugsweise für Halogenid, Hydrogensulfat, ½ Sulfat, Benzolsulfonat, p-Toluolsulfonat, Acetat, Citrat, Lactat, Tetrafluoroborat, Trifluormethansulfonat, Hexafluorophosphat

11. Kationische Derivate nach Anspruch 10, ausgewählt aus
- Salzen des 1,4-Dimethyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Diethyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Diallyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{methyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dibenzyl-5-[(4-{propyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-methyl-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-methoxy-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(2-chlor-4-{ethyl[2-(4-aza-1-azoniabicyclo[2.2.2]octan-1-yl)ethyl]amino}phenyl)diazenyl]-4H-1,2,4-triazol-1-iums
- Salzen des 1,4-Dimethyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Dimethyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Dimethyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Diethyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Diallyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{methyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{ethyl[3-(4-aza-1-azoniabicyclo[2.2.2]octan-1-yl)propyl]amino}phenyl)diazenyl]-4H-1,2,4-triazol-1-iums
- Salzen des 1,4-Dibenzyl-5-[(4-{propyl[3-(4-aza-1-azoniabicyclo[2.2.2]octan-1-yl)propyl]amino}phenyl)diazenyl]-4H-1,2,4-triazol-1-iums
- Salzen des 1,3-Dimethyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Diethyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Diallyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{methyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{ethyl[2-(4-aza-1-azoniabicyclo[2.2.2]octan-1-yl)ethyl]amino}phenyl)diazenyl]-1H-imidazol-3-iums
- Salzen des 1,3-Dibenzyl-2-[(4-{propyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-methyl-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-methoxy-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(2-chlor-4-{ethyl[2-(4-aza-1-azoniabicyclo[2.2.2]octan-1-yl)ethyl]amino}phenyl)diazenyl]-1H-imidazol-3-iums
- Salzen des 1,3-Dimethyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Dimethyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Dimethyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Diethyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Diallyl-2-[(4-{propyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{methyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{ethyl[3-(4-aza-1-azoniabicyclo[2.2.2]octan-1-yl)propyl]amino}phenyl)diazenyl]-1H-imidazol-3-iums
- Salzen des 1,3-Dibenzyl-2-[(4-{propyl[3-(4-aza-1-azoniabicyclo[2.2.2]octan-1-yl)propyl]amino}phenyl)diazenyl]-1H-imidazol-3-iums

## Claims

1. Agent for dyeing and optionally at the same time lightening keratin fibers, in particular human hair, containing at least one dicationic azo dye of formula (I): 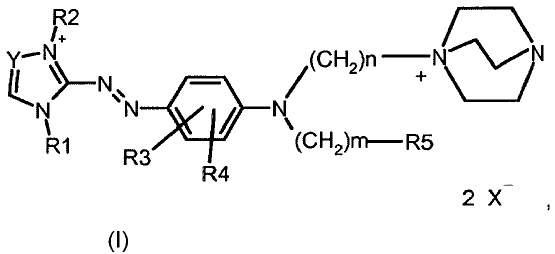 in which
Y stands either for =CH- or a nitrogen atom,
R¹, R² independently stand for a C₁-C₆ alkyl group, a C₂-C₆ alkenyl group, a hydroxy-C₁-C₆ alkyl group, a polyhydroxy-C₂-C₆ alkyl group, a C₁-C₆ alkoxy-C₂-C₆ alkyl group, a cyano-C₁-C₆ alkyl group, an aryl group, or an aryl-C₁-C₆ alkyl group,
R³, R⁴ independently stand for a hydrogen atom, a C₁-C₆ alkyl group, a halogen atom (fluorine, chlorine, bromine, or iodine), a C₁-C₆ alkoxy group, an amino group, a C₁-C₆ alkylamino group, a di-C₁-C₆ alkylamino group, a hydroxy group, or R³ and R⁴ together form a 5-, 6-, or 7-membered saturated or unsaturated ring which may optionally contain further heteroatoms or bear further substituents,
n, m independently stand for an integer from 1 to 6,
R⁵ stands for a hydrogen atom, a C₁-C₆ alkoxy group, a halogen atom (fluorine, chlorine, bromine, or iodine), a hydroxy group, a C₁-C₆ alkoxy-C₂-C₆ alkyloxy group, a nitrile group, an aryl group, or a heteroaryl group,
X- stands for a monovalent anion, preferably for halide, hydrogen sulfate, ½ sulfate, benzenesulfonate, *p*-toluenesulfonate, acetate, citrate, lactate, tetrafluoroborate, trifluoromethanesulfonate, hexafluorophosphate.

2. Agent for dyeing and optionally at the same time lightening keratinic fibers according to Claim 1, **characterized in that** the agent contains compounds of formula (I) in which the radicals R¹ and R² stand for a C₁-C₆ alkyl group (in particular a methyl or ethyl group), a C₂-C₆ alkenyl group (in particular an allyl group), or an aryl-C₁-C₆ alkyl group (in particular a benzyl group).

3. Agent for dyeing and optionally at the same time lightening keratinic fibers according to one of Claims 1 to 2, **characterized in that** the agent contains at least one cationic compound from the group of dicationic azo dyes comprising
- salts of 1,4-dimethyl-5-[(4-{methyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(4-{propyl[2-(4-aza-1-azoniabicyclo[2.2.2]octan-1-yl)ethyl]amino}phenyl)diazenyl]-4H-1,2,4-triazol-1-ium
- salts of 1,4-diethyl-5-[(4-{methyl[2-(4-aza-1-azoniabicyclo[2.2.2]octan-1-yl)ethyl]amino}phenyl)diazenyl]-4H-1,2,4-triazol-1 -ium
- salts of 1,4-diethyl-5-[(4-{ethyl[2-(4-aza-1-azoniabicyclo[2.2.2]octan-1-yl)ethyl]amino}phenyl)diazenyl]-4/-/-1,2,4-triazol-1 -ium
- salts of 1,4-diethyl-5-[(4-{propyl[2-(4-aza-1-azoniabicyclo[2.2.2]octan-1-yl)ethyl]amino}phenyl)diazenyl]-4*H*-1,2,4-triazol-1-ium
- salts of 1,4-diallyl-5-[(4-{methyl[2-(4-aza-1-azoniabicyclo[2.2.2]octan-1-yl)ethyl]amino}phenyl)diazenyl]-4*H*-1,2,4-triazol-1-ium
- salts of 1,4-diallyl-5-[(4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-diallyl-5-[(4-{propyl[2-(4-aza-1-azoniabicyclo[2.2.2]octan-1-yl)ethyl]amino}phenyl)diazenyl]-4*H*-1,2,4-triazol-1-ium
- salts of 1,4-dibenzyl-5-[(4-{methyl[2-(4-aza-1-azoniabicyclo[2.2.2]octan-1-yl)ethyl]amino}phenyl)diazenyl]-4*H*-1,2,4-triazol-1-ium
- salts of 1,4-dibenzyl-5-[(4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dibenzyl-5-[(4-{propyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(2-methyl-4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(2-methoxy-4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(2-chloro-4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(4-{methyl[3-(4-aza-1-azoniabicyclo[2.2.2]octan-1-yl)propyl]amino}phenyl)diazenyl]-4*H*-1,2,4-triazol-1-ium
- salts of 1,4-dimethyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-dimethyl-5-[(4-{propyl[3-(4-aza-1-azoniabicyclo[2.2.2]octan-1-yl)propyl]amino}phenyl)diazenyl]-4*H*-1,2,4-triazol-1-ium
- salts of 1,4-diethyl-5-[(4-{methyl[3-(4-aza-1-azoniabicyclo[2.2.2]octan-1-yl)propyl]amino}phenyl)diazenyl]-4*H*-1,2,4-triazol-1-ium
- salts of 1,4-diethyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-diethyl-5-[(4-{propyl[3-(4-aza-1-azoniabicyclo[2.2.2]octan-1-yl)propyl]amino}phenyl)diazenyl]-4*H*-1,2,4-triazol-1-ium
- salts of 1,4-diallyl-5-[(4-{methyl[3-(4-aza-1-azoniabicyclo[2.2.2]octan-1-yl)propyl]amino}phenyl)diazenyl]-4H-1,2,4-triazol-1-ium
- salts of 1,4-diallyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-diallyl-5-[(4-{propyl[3-(4-aza-1-azoniabicyclo[2.2.2]octan-1-yl)propyl]amino}phenyl)diazenyl]-4*H*-1,2,4-triazol-1-ium
- salts of 1,4-dibenzyl-5-[(4-{methyl[3-(4-aza-1-azoniabicyclo[2.2.2]octan-1-yl)propyl]amino}phenyl)diazenyl]-4*H*-1,2,4-triazol-1-ium
- salts of 1,4-dibenzyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-dibenzyl-5-[(4-{propyl[3-(4-aza-1-azoniabicyclo[2.2.2]octan-1-yl)propyl]amino}phenyl)diazenyl]-4*H*-1,2,4-triazol-1-ium.

4. Agent for dyeing and optionally at the same time lightening keratinic fibers according to one of Claims 1 to 3, **characterized in that** the agent contains at least one cationic compound from the group of dicationic azo dyes comprising
- salts of 1,3-dimethyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(4-{ethyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(4-{propyl[2-(4-aza-1-azoniabicyclo[2.2.2]octan-1-yl)ethyl]amino}phenyl)diazenyl]-1*H*-imidazol-3-ium
- salts of 1,3-diethyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-diethyl-2-[(4-{ethyl[2-(4-aza-1-azoniabicyclo[2.2.2]octan-1-yl)ethyl]amino}phenyl)diazenyl]-1*H*-imidazol-3-ium
- salts of 1,3-diethyl-2-[(4-{propyl[2-(4-aza-1-azoniabicyclo[2.2.2]octan-1-yl)ethyl]amino}phenyl)diazenyl]-1 H-imidazol-3-ium
- salts of 1,3-diallyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-diallyl-2-[(4-{ethyl[2-(4-aza-1 -azoniabicyclo[2.2.2]octan-1-yl)ethyl]amino}phenyl)diazenyl]-1*H*-imidazol-3-ium
- salts of 1,3-diallyl-2-[(4-{propyl[2-(4-aza-1-azoniabicyclo[2.2.2]octan-1-yl)ethyl]amino}phenyl)diazenyl]-1*H*-imidazol-3-ium
- salts of 1,3-dibenzyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-dibenzyl-2-[(4-{ethyl[2-(4-aza-1-azoniabicyclo[2.2.2]octan-1-yl)ethyl]amino}phenyl)diazenyl]-1*H*-imidazol-3-ium
- salts of 1,3-dibenzyl-2-[(4-{propyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(2-methyl-4-{ethyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(2-methoxy-4-{ethyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(2-chloro-4-{ethyl[2-(4-aza-1-azoniabicyclo[2.2.2]octan-1 yl)ethyl]amino}phenyl)diazenyl]-1*H*-imidazol-3-ium
- salts of 1,3-dimethyl-2-[(4-{methyl[3-(4-aza-1-azoniabicyclo[2.2.2]octan-1-yl)propyl]amino}phenyl)diazenyl]-1*H*-imidazol-3-ium
- salts of 1,3-dimethyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-dimethyl-2-[(4-{propyl[3-(4-aza-1-azoniabicyclo[2.2.2]octan-1-yl)propyl]amino}phenyl)diazenyl]-1*H*-imidazol-3-ium
- salts of 1,3-diethyl-2-[(4-{methyl[3-(4-aza-1-azoniabicyclo[2.2.2]octan-1-yl)propyl]amino}phenyl)diazenyl]-1*H*-imidazol-3-ium
- salts of 1,3-diethyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-diethyl-2-[(4-{propyl[3-(4-aza-1-azoniabicyclo[2.2.2]octan-1-yl)propyl]amino}phenyl)diazenyl]-1*H*-imidazol-3-ium
- salts of 1,3-diallyl-2-[(4-{methyl[3-(4-aza-1-azoniabicyclo[2.2.2]octan-1-yl)propyl]amino}phenyl)diazenyl]-1*H*-imidazol-3-ium
- salts of 1,3-diallyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-diallyl-2-[(4-{propyl[3-(4-aza-1-azoniabicyclo[2.2.2]octan-1-yl)propyl]amino}phenyl)diazenyl]-1*H*-imidazol-3-ium
- salts of 1,3-dibenzyl-2-[(4-{methyl[3-(4-aza-1-azoniabicyclo[2.2.2]octan-1 yl)propyl]amino}phenyl)diazenyl]-1*H*-imidazol-3-ium
- salts of 1,3-dibenzyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-dibenzyl-2-[(4-{propyl[3-(4-aza-1-azoniabicyclo[2.2.2]octan-1-yl)propyl]amino}phenyl)diazenyl]-1*H*-imidazol-3-ium

5. Agent for dyeing and optionally at the same time lightening keratinic fibers according to one of Claims 1 to 4, **characterized in that** the agent contains the compound(s) of formula (I) in quantities of 0.001 to 5% by weight, preferably 0.0025 to 2.5% by weight, particularly preferably 0.005 to 1.5% by weight, and in particular 0.01 to 1% by weight, in each case based on the overall agent.

6. Agent for dyeing and optionally at the same time lightening keratinic fibers according to one of Claims 1 to 5, **characterized in that** the agent additionally contains, based on its weight, 0.001 to 5% by weight of one or more oxidation dye precursors and/or direct dyes.

7. Agent for dyeing and optionally at the same time lightening keratinic fibers according to one of Claims 1 to 6, **characterized in that** the agent contains 0.5 to 15% by weight, preferably 1 to 12.5% by weight, particularly preferably 2.5 to 10% by weight, and in particular 3 to 6% by weight, of hydrogen peroxide (calculated as 100% H₂O₂).

8. Method for dyeing and optionally at the same time lightening keratin fibers, in particular human hair, **characterized in that**
- a pretreatment agent M1 is applied, if desired, to the fiber, then
- an agent M2 is applied to the fiber, a further agent M3 being added, if desired, to agent M2 prior to application,
- this agent M2 is rinsed from the fiber after a period of 5-30 minutes, and
- after the treatment, a post-treatment agent M4 is optionally applied to the
fiber and is rinsed off after an exposure period of several minutes,
wherein at least one of the agents M1, M2, or M3 is an agent according to one of Claims 1 to 6.

9. Use of dicationic azo dyes of formula (I): 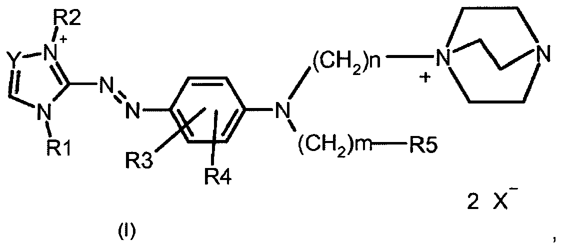 in which
Y stands either for =CH- or a nitrogen atom,
R¹, R² independently stand for a C₁-C₆ alkyl group, a C₂-C₆ alkenyl group, a hydroxy-C₁-C₆ alkyl group, a polyhydroxy-C₂-C₆ alkyl group, a C₁-C₆ alkoxy-C₂-C₆ alkyl group, a cyano-C₁-C₆ alkyl group, an aryl group, or an aryl-C₁-C₆ alkyl group,
R³, R⁴ independently stand for a hydrogen atom, a C₁-C₆ alkyl group, a halogen atom (fluorine, chlorine, bromine, or iodine), a C₁-C₆ alkoxy group, an amino group, a C₁-C₆ alkylamino group, a di-C₁-C₆ alkylamino group, a hydroxy group, or R³ and R⁴ together form a 5-, 6-, or 7-membered saturated or unsaturated ring which may optionally contain further heteroatoms or bear further substituents,
n, m independently stand for an integer from 1 to 6,
R⁵ stands for a hydrogen atom, a C₁-C₆ alkoxy group, a halogen atom (fluorine, chlorine, bromine, or iodine), a hydroxy group, a C₁-C₆ alkoxy-C₂-C₆ alkyloxy group, a nitrile group, an aryl group, or a heteroaryl group,
X- stands for a monovalent anion, preferably for halide, hydrogen sulfate, ½ sulfate, benzenesulfonate, *p*-toluenesulfonate, acetate, citrate, lactate, tetrafluoroborate, trifluoromethanesulfonate, hexafluorophosphate
in agents for dyeing and optionally at the same time lightening keratinic fibers.

10. Dicationic azo dyes of formula (I): 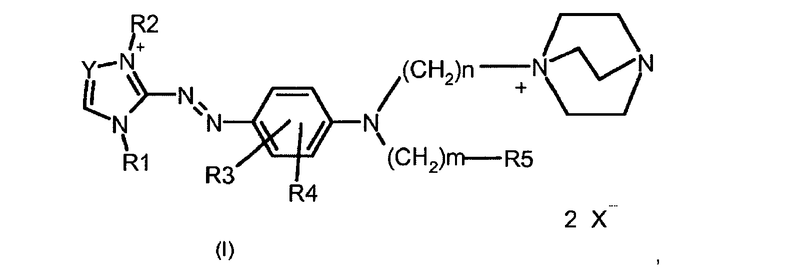 in which
Y stands either for =CH- or a nitrogen atom,
R¹, R² independently stand for a C₁-C₆ alkyl group, a C₂-C₆ alkenyl group, a hydroxy-C₁-C₆ alkyl group, a polyhydroxy-C₂-C₆ alkyl group, a C₁-C₆ alkoxy-C₂-C₆ alkyl group, a cyano-C₁-C₆ alkyl group, an aryl group, or an aryl-C₁-C₆ alkyl group,
R³, R⁴ independently stand for a hydrogen atom, a C₁-C₆ alkyl group, a halogen atom (fluorine, chlorine, bromine, or iodine), a C₁-C₆ alkoxy group, an amino group, a C₁-C₆ alkylamino group, a di-C₁-C₆ alkylamino group, a hydroxy group, or R³ and R⁴ together form a 5-, 6-, or 7-membered saturated or unsaturated ring which may optionally contain further heteroatoms or bear further substituents,
n, m independently stand for an integer from 1 to 6,
R⁵ stands for a hydrogen atom, a C₁-C₆ alkoxy group, a halogen atom (fluorine, chlorine, bromine, or iodine), a hydroxy group, a C₁-C₆ alkoxy-C₂-C₆ alkyloxy group, a nitrile group, an aryl group, or a heteroaryl group,
X- stands for a monovalent anion, preferably for halide, hydrogen sulfate, ½ sulfate, benzenesulfonate, *p*-toluenesulfonate, acetate, citrate, lactate, tetrafluoroborate, trifluoromethanesulfonate, hexafluorophosphate.

11. Cationic derivatives according to Claim 10, selected from
- salts of 1,4-dimethyl-5-[(4-{methyl[2-(4-aza-1-azoniabicyclo[2.2.2]octan-1-yl)ethyl]amino }phenyl)diazenyl]-4*H*-1,2,4-triazol-1-ium
- salts of 1,4-dimethyl-5-[(4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(4-{propyl[2-(4-aza-1-azoniabicyclo[2.2.2]octan-1-yl)ethyl]amino}phenyl)diazenyl]-4*H*-1,2,4-triazol-1-ium
- salts of 1,4-diethyl-5-[(4-{methyl[2-(4-aza-1-azoniabicyclo[2.2.2]octan-1-yl)ethyl]amino}phenyl)diazenyl]-4*H*-1,2,4-triazol-1-ium
- salts of 1,4-diethyl-5-[(4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-diethyl-5-[(4-{propyl[2-(4-aza-1-azoniabicyclo[2.2.2]octan-1-yl)ethyl]amino}phenyl)diazenyl]-4*H*-1,2,4-triazol-1-ium
- salts of 1,4-diallyl-5-[(4-{methyl[2-(4-aza-1-azoniabicyclo[2.2.2]octan-1-yl)ethyl]amino}phenyl)diazenyl]-4*H*-1,2,4-triazol-1-ium
- salts of 1,4-diallyl-5-[(4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-diallyl-5-[(4-{propyl[2-(4-aza-1-azoniabicyclo[2.2.2]octan-1-yl)ethyl]amino}phenyl)diazenyl]-4*H*-1,2,4-triazol-1-ium
- salts of 1,4-dibenzyl-5-[(4-{methyl[2-(4-aza-1-azoniabicyclo[2.2.2]octan-1-yl)ethyl]amino}phenyl)diazenyl]-4*H*-1,2,4-triazol-1-ium
- salts of 1,4-dibenzyl-5-[(4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dibenzyl-5-[(4-{propyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(2-methyl-4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(2-methoxy-4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(2-chloro-4-{ethyl[2-(4-aza-1-azoniabicyclo[2.2.2]octan-1-yl)ethyl]amino}phenyl)diazenyl]-4*H*-1,2,4-triazol-1-ium
- salts of 1,4-dimethyl-5-[(4-{methyl[3-(4-aza-1-azoniabicyclo[2.2.2]octan-1-yl)propyl]amino}phenyl)diazenyl]-4*H*-1,2,4-triazol-1-ium
- salts of 1,4-dimethyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-dimethyl-5-[(4-{propyl[3-(4-aza-1-azoniabicyclo[2.2.2]octan-1-yl)propyl]amino}phenyl)diazenyl]-4*H*-1,2,4-triazol-1-ium
- salts of 1,4-diethyl-5-[(4-{methyl[3-(4-aza-1-azoniabicyclo[2.2.2]octan-1-yl)propyl]amino}phenyl)diazenyl]-4*H*-1,2,4-triazol-1-ium
- salts of 1,4-diethyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-diethyl-5-[(4-{propyl[3-(4-aza-1-azoniabicyclo[2.2.2]octan-1-yl)propyl]amino}phenyl)diazenyl]-4*H*-1,2,4-triazol-1-ium
- salts of 1,4-diallyl-5-[(4-{methyl[3-(4-aza-1-azoniabicyclo[2.2.2]octan-1-yl)propyl]amino}phenyl)diazenyl]-4*H*-1,2,4-triazol-1-ium
- salts of 1,4-diallyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-diallyl-5-[(4-{propyl[3-(4-aza-1-azoniabicyclo[2.2.2]octan-1-yl)propyl]amino}phenyl)diazenyl]-4*H*-1,2,4-triazol-1-ium
- salts of 1,4-dibenzyl-5-[(4-{methyl[3-(4-aza-1-azoniabicyclo[2.2.2]octan-1-yl)propyl]amino}phenyl)diazenyl]-4*H*-1,2,4-triazol-1-ium
- salts of 1,4-dibenzyl-5-[(4-{ethyl[3-(4-aza-1-azoniabicyclo[2.2.2]octan-1-yl)propyl]amino}phenyl)diazenyl]-4*H*-1,2,4-triazol-1-ium
- salts of 1,4-dibenzyl-5-[(4-{propyl[3-(4-aza-1-azoniabicyclo[2.2.2]octan-1-yl)propyl]amino}phenyl)diazenyl]-4*H*-1,2,4-triazol-1-ium
- salts of 1,3-dimethyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(4-{ethyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(4-{propyl[2-(4-aza-1-azoniabicyclo[2.2.2]octan-1-yl)ethyl]amino}phenyl)diazenyl]-1*H*-imidazol-3-ium
- salts of 1,3-diethyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-diethyl-2-[(4-{ethyl[2-(4-aza-1-azoniabicyclo[2.2.2]octan-1-yl)ethyl]amino}phenyl)diazenyl]-1*H*-imidazol-3-ium
- salts of 1,3-diethyl-2-[(4-{propyl[2-(4-aza-1-azoniabicyclo[2.2.2]octan-1-yl)ethyl]amino}phenyl)diazenyl]-1*H*-imidazol-3-ium
- salts of 1,3-diallyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-diallyl-2-[(4-{ethyl[2-(4-aza-1-azoniabicyclo[2.2.2]octan-1-yl)ethyl]amino}phenyl)diazenyl]-1*H*-imidazol-3-ium
- salts of 1,3-diallyl-2-[(4-{propyl[2-(4-aza-1-azoniabicyclo[2.2.2]octan-1-yl)ethyl]amino}phenyl)diazenyl]-1*H*-imidazol-3-ium
- salts of 1,3-dibenzyl-2-[(4-{methyl[2-(4-aza-1-azoniabicyclo[2.2.2]octan-1-yl)ethyl]amino}phenyl)diazenyl]-1*H*-imidazol-3-ium
- salts of 1,3-dibenzyl-2-[(4-{ethyl[2-(4-aza-1-azoniabicyclo[2.2.2]octan-1-yl)ethyl]amino}phenyl)diazenyl]-1*H*-imidazol-3-ium
- salts of 1,3-dibenzyl-2-[(4-{propyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(2-methyl-4-{ethyl[2-(4-aza-1-azoniabicyclo[2.2.2]octan-1-yl)ethyl]amino}phenyl)diazenyl]-1 H-imidazol-3-ium
- salts of 1,3-dimethyl-2-[(2-methoxy-4-{ethyl[2-(4-aza-1-azoniabicyclo[2.2.2]octan-1-yl)ethyl]amino}phenyl)diazenyl]-1*H*-imidazol-3-ium
- salts of 1,3-dimethyl-2-[(2-chloro-4-{ethyl[2-(4-aza-1-azoniabicyclo[2.2.2]octan-1-yl)ethyl]amino}phenyl)diazenyl]-1 H-imidazol-3-ium
- salts of 1,3-dimethyl-2-[(4-{methyl[3-(4-aza-1-azoniabicyclo[2.2.2]octan-1-yl)propyl]amino}phenyl)diazenyl]-1*H*-imidazol-3-ium
- salts of 1,3-dimethyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-dimethyl-2-[(4-{propyl[3-(4-aza-1-azoniabicyclo[2.2.2]octan-1-yl)propyl]amino}phenyl)diazenyl]-1*H*-imidazol-3-ium
- salts of 1,3-diethyl-2-[(4-{methyl[3-(4-aza-1-azoniabicyclo[2.2.2]octan-1-yl)propyl]amino}phenyl)diazenyl]-1*H*-imidazol-3-ium
- salts of 1,3-diethyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-diethyl-2-[(4-{propyl[3-(4-aza-1-azoniabicyclo[2.2.2]octan-1-yl)propyl]amino}phenyl)diazenyl]-1*H*-imidazol-3-ium
- salts of 1,3-diallyl-2-[(4-{methyl[3-(4-aza-1-azoniabicyclo[2.2.2]octan-1-yl)propyl]amino}phenyl)diazenyl]-1*H*-imidazol-3-ium
- salts of 1,3-diallyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-diallyl-2-[(4-{propyl[3-(4-aza-1-azoniabicyclo[2.2.2]octan-1-yl)propyl]amino}phenyl)diazenyl]-1*H*-imidazol-3-ium
- salts of 1,3-dibenzyl-2-[(4-{methyl[3-(4-aza-1-azoniabicyclo[2.2.2]octan-1-yl)propyl]amino}phenyl)diazenyl]-1*H*-imidazol-3-ium
- salts of 1,3-dibenzyl-2-[(4-{ethyl[3-(4-aza-1-azoniabicyclo[2.2.2]octan-1-yl)propyl]amino}phenyl)diazenyl]-1*H*-imidazol-3-ium
- salts of 1,3-dibenzyl-2-[(4-{propyl[3-(4-aza-1-azoniabicyclo[2.2.2]octan-1-yl)propyl]amino}phenyl)diazenyl]-1*H*-imidazol-3-ium.

## Revendications

1. Composition pour la coloration et, éventuellement l'éclaircissement simultané, de fibres de kératine, notamment des cheveux humains, contenant au moins un colorant azoïque dicationique de la formule (I) : 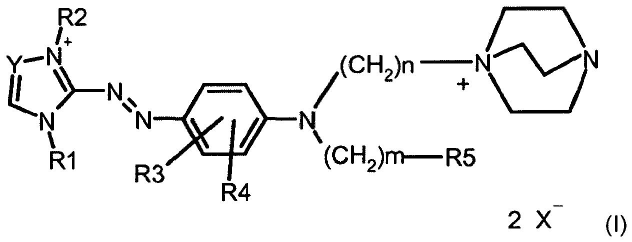 dans laquelle
Y représente soit =CH- soit un atome d'azote,
R¹, R² représentent indépendamment un groupe alkyl en C₁ à C₆, un groupe alcényl en C₂ à C₆, un groupe hydroxy-alkyl en C₁ à C₆, un groupe polyhydroxy-alkyl en C₂ à C₆, un groupe alcoxy en C₁ à C₆-alkyl en C₂ à C₆, un groupe cyano-alkyl en C₁ à C₆, un groupe aryl ou un groupe aryl-alkyl en C₁ à C₆,
R³, R⁴ représentent indépendamment un atome d'hydrogène, un groupe alkyl en C₁ à C₆, un atome d'halogène (fluor, chlore, brome ou iode), un groupe alcoxy en C₁ à C₆, un groupe amino, un groupe alkylamino en C₁ à C₆, un groupe di-alkyl en C₁ à C₆-amino, un groupe hydroxy, ou bien R³ et R⁴ forment conjointement un cycle saturé ou insaturé à 5, 6 ou 7 chaînons, qui, éventuellement, peut contenir d'autres hétéroatomes ou porter d'autres substituants,
n, m représentent indépendamment un nombre entier de 1 à 6,
R⁵ représente un atome d'hydrogène, un groupe alcoxy en C₁ à C₆, un atome d'halogène (fluor, chlore, brome ou iode), un groupe hydroxy, un groupe alcoxy en C₁ à C₆-alkyloxy en C₂ à C₆, un groupe nitrile, un groupe aryle ou un groupe hétéroaryle,
X- représente un anion monovalent, de préférence un halogénure, un hydrogénosulfate, un ½ sulfate, un benzène-sulfonate, un p-toluènesulfonate, un acétate, un citrate, un lactate, un tétrafluoroborate, un trifluorométhane-sulfonate, un hexafluorophosphate.

2. Composition pour la coloration et éventuellement l'éclaircissement simultané de fibres de kératine selon la revendication 1, **caractérisée en ce qu'**elle contient des composés de la formule (I), dans laquelle les radicaux R¹ et R² représentent un groupe alkyl en C₁ à C₆ (notamment un groupe méthyl ou éthyl), un groupe alcényl en C₂ à C₆ (notamment un groupe allyl) ou un groupe aryl-alkyl en C₁ à C₆ (notamment un groupe benzyl).

3. Composition pour la coloration et éventuellement l'éclaircissement simultané de fibres de kératine selon l'une des revendications 1 à 2, **caractérisée en ce qu'**elle contient au moins un composé cationique choisi dans le groupe des colorants azoïques dicationique qui est formé par
- les sels du 1,4-diméthyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(4-{propyl[2-(4-aza-1-azoniabicyclo[2.2.2]octan-1-yl)éthyl]amino}phényl)diazényl]-4H-1,2,4-triazol-1-ium
- les sels du 1,4-diéthyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-diéthyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-diéthyl-5-[(4-{propyl[2-(4-aza-1-azoniabicyclo[2.2.2]octan-1-yl)éthyl]amino}phényl)diazényl]-4H-1,2,4-triazol-1-ium
- les sels du 1,4-diallyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-diallyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-diallyl-5-[(4-{propyl[2-(4-aza-1-azoniabicyclo[2-2.2]octan-1-yl)éthyl]amino}phényl)diazényl]-4H-1,2,4-triazol-1-ium
- les sels du 1,4-dibenzyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-dibenzyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-dibenzyl-5-[(4-{propyl[2-(4-aza-1-azoniabicyclo[2.2.2]octan-1-yl)éthyl]amino}phenyl)diazényl]-4H-1,2,4-triazol-1-ium
- les sels du 1(4-diméthyl-5-[(2-méthyl-4-{éthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(2-méthoxy-4-{éthyl[2-(4-aza-1-azoniabicyclo[22.2]octan-1-yl)éthyl]amino }phényl)diazényl]-4H-1 ,2,4-triazol-1-ium
- les sels du 1,4-diméthyl-5-[(2-chloro-4-{éthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(4-{méthyl[3-(4-aza-1 -azoniabicyclo[2.2.2]octan-1-yl)propyl]amino}phényl)diazényl]-4H-1,2,4-triazol-1-ium
- les sels du 1,4-diméthyl-5-[(4-{éthyl[3-(4-aza-1-azoniabicyclo[2.2.2]octan-1-yl)propyl]amino}phényl)diazényl]-4H-1,2,4-triazol-1-ium
- les sels du 1,4-diméthyl-5-[(4-{propyl[3-(4-aza-1-azoniabicyclo[2.2.2]octan-1-yl)propyl]amino}phényl)diazényl]-4H-1,2,4-triazol-1-ium
- les sels du 1,4-diéthyl-5-[(4-{méthyl[3-(4-aza-1-azoniabicyclo[2.2.2]octan-1-yl)propyl]amino}phényl)diazényl]-4H-1,2,4-triazol-1-ium
- les sels du 1,4-diéthyl-5-[(4-{éthyl[3-(4-aza-1-azoniabicyclo[2.2.2]octan-1-yl)propyl]amino}phényl)diazényl]-4H-1,2,4-triazol-1-ium
- les sels du 1,4-diéthyl-5-[(4-{propyl[3-(4-aza-1-azoniabicyclo[2.2.2]octan-1-yl)propyl]amino}phényl)diazényl]-4H-1,2,4-triazol-1-ium
- les sels du 1,4-diallyl-5-[(4-{méthyl[3-(4-aza-1-azoniabicyclo[2.2.2]octan-1-yl)propyl]amino}phényl)diazényl]-4H-1,2,4-triazol-1-ium
- les sels du 1,4-diallyl-5-[(4-{éthyl[3-(4-aza-1-azoniabicyclo[2.2.2]octan-1-yl)propyl]amino}phényl)diazényl]-4H-1,2,4-triazol-1-ium
- les sels du 1,4-diallyl-5-[(4-{propyl[3-(4-aza-1-azoniabicyclo[2.2.2]octan-1-yl)propyl]amino}phényl)diazényl]-4H-1,2,4-triazol-1-ium
- les sels du 1,4-dibenzyl-5-[(4-{méthyl[3-(4-aza-1-azoniabicyclo[2.2.2]octan-1-yl)propyl]amino}phényl)diazényl]-4H-1,2,4-triazol-1-ium
- les sels du 1,4-dibenzyl-5-[(4-{éthyl[3-(4-aza-1-azoniabicyclo[2.2.2]octan-1-yl)propyl]amino}phényl)diazényl]-4H-1,2,4-triazol-1-ium
- les sels du 1,4-dibenzyl-5-[(4-{propyl[3-(4-aza-1-azoniabicyclo[2.2.2]octan-1-yl)propyl]amino}phényl)diazényl]-4H-1 (2,4-triazol-1-ium.

4. Composition pour la coloration et éventuellement l'éclaircissement simultané des fibres de kératine selon l'une des revendications 1 à 3, **caractérisée en ce qu'**elle contient au moins un composé cationique choisi dans le groupe des colorants azoïques dicationiques qui est formé par
- les sels du 1,3-diméthyl-2-[(4-{méthyl[2-(4-aza-1-azoniabicyclo[2.2.2]octan-1-yl)éthyl]amino}phényl)diazényl]-1H-imidazol-3-ium
- les sels du 1,3-diméthyl-2-[(4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(4-{propyl[2-(4-aza-1-azoniabicyclo[2.2.2]octan-1-yl)éthyl]amino}phényl)diazényl]-1H-imidazol-3-ium
- les sels du 1,3-diéthyl-2-[(4-{méthyl[2-(4-aza-1-azoniabicyclo[2.2.2]octan-1-yl)éthyl]amino}phényl)diazényl]-1H-imidazol-3-ium
- les sels du 1,3-diéthyl-2-[(4-{éthyl[2-(4-aza-1-azoniabicyclo[2.2.2]octan-1-yl)éthyl]amino}phényl)diazényl]-1H-imidazol-3-ium
- les sels du 1,3-diéthyl-2-[(4-{propyl[2-(4-aza-1-azoniabicyclo[2.2.2]octan-1-yl)éthyl]amino}phényl)diazényl]-1H-imidazol-3-ium
- les sels du 1,3-diallyl-2-[(4-{méthyl[2-(4-aza-1-azoniabicyclo[2.2.2]octan-1-yl)éthyl]amino}phényl)diazényl]-1H-imidazol-3-ium
- les sels du 1,3-diallyl-2-[(4-{éthyl[2-(4-aza-1-azoniabicyclo[2.2.2]octan-1-yl)éthyl]amino}phényl)diazényl]-1H-imidazol-3-ium
- les sels du 1,3-diallyl-2-[(4-{propyl[2-(4-aza-1-azoniabicyclo[2.2.2]octan-1-yl)éthyl]amino}phényl)diazényl]-1H-imidazol-3-ium
- les sels du 1,3-dibenzyl-2-[(4-{méthyl[2-(4-aza-1-azoniabicyclo[2.2.2]octan-1-yl)éthyl]amino}phényl)diazényl]-1H-imidazol-3-ium
- les sels du 1,3-dibenzyl-2-[(4-{éthyl[2-(4-aza-1-azoniabicyclo[2.2.2]octan-1-yl)éthyl]amino}phényl)diazényl]-1H-imidazol-3-ium
- les sels du 1,3-dibenzyl-2-[(4-{propyl[2-(4-aza-1-azoniabicyclo[2.2.2]octan-1-yl)éthyl]amino}phényl)diazényl]-1H-imidazol-3-ium
- les sels du 1,3-diméthyl-2-(2-méthyl-4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(2-méthoxy-4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(2-chloro-4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(4-{méthyl[3-(4-aza-1-azoniabicyclo[2.2.2]octan-1-yl)propyl]amino}phényl)diazényl]-1H-imidazol-3-ium
- les sels du 1,3-diméthyl-2-[(4-{éthyl[3-(4-aza-1-azoniabicyclo[2.2.2]octan-1-yl)propyl]amino}phényl)diazényl]-1H-imidazol-3-ium
- les sels du 1,3-diméthyl-2-[(4-{propyl[3-(4-aza-1-azoniabicyclo[2.2.2]octan-1-yl)propyl]amino}phényl)diazényl]-1H-imidazol-3-ium
- les sels du 1,3-diéthyl-2-[(4-{méthyl[3-(4-aza-1-azoniabicyclo[2.2.2]octan-1-yl)propyl]amino}phényl)diazényl]-1H-imidazol-3-ium
- les sels du 1,3-diéthyl-2-[(4-{éthyl[3-(4-aza-1-azoniabicyclo[2.2.2]octan-1-yl)propyl]amino}phényl)diazényl]-1H-imidazol-3-ium
- les sels du 1,3-diéthyl-2-[(4-{propyl[3-(4-aza-1-azoniabicyclo[2.2.2]octan-1-yl)propyl]amino}phényl)diazényl]-1H-imidazol-3-ium
- les sels du 1,3-diallyl-2-(4-{méthyl[3-(4-aza-1-azoniabicyclo[2.2.2]octan-1-yl)propyl]amino}phényl)diazényl]-1H-imidazol-3-ium
- les sels du 1,3-dially!-2-[(4-{éthyl[3-(4-aza-1-azoniabicyclo[2.2.2]octan-1-yl)propyl]amino}phényl)diazényl]-1H-imidazol-3-ium
- les sels du 1,3-diallyl-2-[(4-{propyl[3-(4-aza-1-azoniabicyclo[2.2.2]octan-1-yl)propyl]amino}phényl)diazényl]-1H-imidazol-3-ium
- les sels du 1,3-dibenzyl-2-[(4-{méthyl[3-(4-aza-1-azoniabicyclo[2.2.2]octan-1-yl)propyl]amino}phényl)diazényl]-1H-imidazol-3-ium
- les sels du 1,3-dibenzyl-2-[(4-{éthyl[3-(4-aza-1-azoniabicyclo[2.2.2]octan-1-yl)propyl]amino}phényl)diazényl]-1H-imidazol-3-ium
- les sels du 1,3-dibenzyl-2-[(4-{propyl[3-(4-aza-1-azoniabicyclo[2.2.2]octan-1-yl)propyl]amino}phényl)diazényl]-1H-imidazol-3-ium.

5. Composition pour la coloration et éventuellement l'éclaircissement simultané de fibres de kératine selon l'une des revendications 1 à 4, **caractérisée en ce qu'**elle contient le ou les composés de la formule (I) dans des quantités allant de 0,001 à 5% en poids, de préférence de 0,0025 à 2,5% en poids, plus préférablement de 0,005 à 1,5% en poids et notamment de 0,01 à 1% en poids, à chaque fois sur la base de la composition totale.

6. Composition pour la coloration et éventuellement l'éclaircissement simultané de fibres de kératine selon l'une des revendications 1 à 5, **caractérisée en ce qu'**elle contient en outre, sur la base de son poids, 0,001 à 5% en poids d'un ou de plusieurs précurseurs de colorant d'oxydation et/ou de colorants directs.

7. Composition pour la coloration et éventuellement l'éclaircissement simultané de fibres de kératine selon l'une des revendications 1 à 6, **caractérisée en ce qu'**elle contient de 0,5 à 15% en poids, de préférence de 1 à 12,5% en poids, plus préférablement de 2,5 à 10 % en poids et notamment de 3 à 6% en poids de peroxyde d'hydrogène (calculé par rapport à 100% de H₂O₂).

8. Procédé pour la coloration et éventuellement l'éclaircissement simultané de fibres de kératine, notamment de cheveux humains, **caractérisé en ce que**
- une composition de prétraitement M1 est appliquée, si on le souhaite, sur les fibres, puis
- une composition M2 est appliquée sur les fibres, une autre composition M3 étant ajoutée, si on le souhaite, à la composition M2 avant l'application,
- cette composition M2 est éliminée des fibres par rinçage au bout de 5 à 30 minutes,
- et après le traitement, éventuellement une composition de post-traitement M4 est appliquée sur les fibres et de nouveau éliminée par rinçage après avoir laissé agir pendant quelques minutes,
au moins une des compositions M1, M2 ou M3 étant une composition selon l'une quelconque des revendications 1 à 6.

9. Utilisation de colorants azoïques dicationiques de la formule (I) : 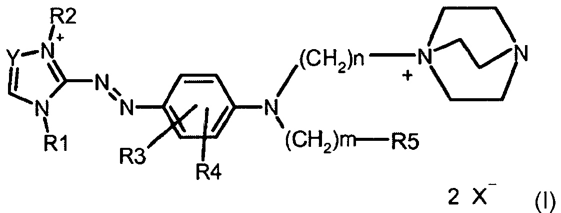 dans laquelle
Y représente soit =CH- soit un atome d'azote,
R¹, R² représentent indépendamment un groupe alkyl en C₁ à C₆, un groupe alcényl en C₂ à C₆, un groupe hydroxy-alkyl en C₁ à C₆, un groupe polyhydroxy-alkyl en C₂ à C₆, un groupe alcoxy en C₁ à C₆-alkyl en C₂ à C₆, un groupe cyano-alkyl en C₁ à C₆, un groupe aryl ou un groupe aryl-alkyl en C₁ à C₆,
R³, R⁴ représentent indépendamment un atome d'hydrogène, un groupe alkyl en C₁ à C₆, un atome d'halogène (fluor, chlore, brome ou iode), un groupe alcoxy en C₁ à C₆, un groupe amino, un groupe alkylamino en C₁ à C₆, un groupe di-alkyl en C₁ à C₆-amino, un groupe hydroxy, ou bien R³ et R⁴ forment conjointement un cycle saturé ou insaturé à 5, 6 ou 7 chaînons, qui, éventuellement, peut contenir d'autres hétéroatomes ou porter d'autres substituants,
n, m représentent indépendamment un nombre entier de 1 à 6,
R⁵ représente un atome d'hydrogène, un groupe alcoxy en C₁ à C₆, un atome d'halogène (fluor, chlore, brome ou iode), un groupe hydroxy, un groupe alcoxy en C₁ à C₆-alkyloxy en C₂ à C₆, un groupe nitrile, un groupe aryle ou un groupe hétéroaryle,
X- représente un anion monovalent, de préférence un halogénure, un hydrogénosulfate, un ½ sulfate, un benzène-sulfonate, un p-toluènesulfonate, un acétate, un citrate, un lactate, un tétrafluoroborate, un trifluorométhane-sulfonate, un hexafluorophosphate
dans des compositions pour la coloration et éventuellement l'éclaircissement simultané de fibres de kératine.

10. Colorants azoïques dicationiques de la formule (I): 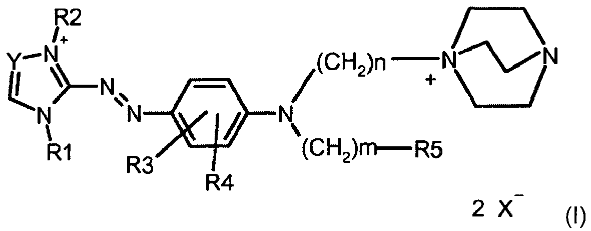 dans laquelle
Y représente soit =CH- soit un atome d'azote,
R¹, R² représentent indépendamment un groupe alkyl en C₁ à C₆, un groupe alcényl en C₂ à C₆, un groupe hydroxy-alkyl en C₁ à C₆, un groupe polyhydroxy-alkyl en C₂ à C₆, un groupe alcoxy en C₁ à C₆-alkyl en C₂ à C₆, un groupe cyano-alkyl en C₁ à C₆, un groupe aryl ou un groupe aryl-alkyl en C₁ à C₆,
R³, R⁴ représentent indépendamment un atome d'hydrogène, un groupe alkyl en C₁ à C₆, un atome d'halogène (fluor, chlore, brome ou iode), un groupe alcoxy en C₁ à C₆, un groupe amino, un groupe alkylamino en C₁ à C₆, un groupe di-alkyl en C₁ à C₆-amino, un groupe hydroxy, ou bien R³ et R⁴ forment conjointement un cycle saturé ou insaturé à 5, 6 ou 7 chaînons, qui, éventuellement, peut contenir d'autres hétéroatomes ou porter d'autres substituants,
n, m représentent indépendamment un nombre entier de 1 à 6,
R⁵ représente un atome d'hydrogène, un groupe alcoxy en C₁ à C₆, un atome d'halogène (fluor, chlore, brome ou iode), un groupe hydroxy, un groupe alcoxy en C₁ à C₆-alkyloxy en C₂ à C₆, un groupe nitrile, un groupe aryle ou un groupe hétéroaryle,
X- représente un anion monovalent, de préférence un halogénure, un hydrogénosulfate, un ½ sulfate, un benzène-sulfonate, un p-toluènesulfonate, un acétate, un citrate, un lactate, un tétrafluoroborate, un trifluorométhane-sulfonate, un hexafluorophosphate.

11. Dérivés cationiques selon la revendication 10, choisis parmi
- les sels du 1,4-diméthyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(4-{propyl[2-(4-aza-1-azoniabicyclo[2.2.2]octan-1-yl)éthyl]amino}phényl)diazényl]-4H-1,2,4-triazol-1-ium
- les sels du 1,4-diéthyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-diéthyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-diéthyl-5-[(4-{propyl[2-(4-aza-1-azoniabicyclo[2.2.2]octan-1-yl)éthyl]amino}phényl)diazényl]-4H-1,2,4-triazol-1-ium
- les sels du 1,4-diallyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-diallyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-diallyl-5-[(4-{propyl[2-(4-aza-1-azoniabicyclo[2.2.2]octan-1-yl)éthyl]amino}phényl)diazényl]-4H-1,2,4-triazol-1-ium
- les sels du 1-4-dibenzyl-5-[(4-{méthyl[2-(4-aza-1-azoniabicyclo[2.2.2]octan-1-yl)éthyl]amino}phényl)diazényl]-4H-1,2,4-triazol-1-ium
- les sels du 1,4-dibenzyl-5-[(4-{éthyl[2-(4-aza-1-azoniabicyclo[2.2.2]octan-1-yl)éthyl]amino}phényl)diazényl]-4H-1,2,4-triazol-1-ium
- les sels du 1,4-dibenzyl-5-[(4-{propyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(2-méthyl-4-{éthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(2-méthoxy-4-{éthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(2-chlor-4-{éthyl[2-(4-aza-1-azoniabicyclo[2.2.2]octan-1-yl)éthyl]amino}phényl)diazényl]-4H-1,2,4-triazol-1-ium
- les sels du 1,4-diméthyl-5-[(4-{méthyl[3-(4-aza-1-azoniabicyclo[2.2.2]octan-1-yl)propyl]amino}phényl)diazényl]-4H-1,2,4-triazol-1-ium
- les sels du 1,4-diméthyl-5-[(4-{éthyl[3-(4-aza-1-azoniabicyclo[2.2.2]octan-1-yl)propyl]aminolphényl)diazényl]-4H-1,2,4-triazol-1-ium
- les sels du 1,4-diméthyl-5-[(4-{propyl[3-(4-aza-1-azoniabicyclo[2.2.2]octan-1-yl)propyl]amino}phényl)diazényl]-4H-1,2,4-triazol-1-ium
- les sels du 1,4-diéthyl-5-[(4-{méthyl[3-(4-aza-1-azoniabicyclo[2.2.2]octan-1-yl)propyl]amino}phényl)diazényl]-4H-1,2,4-triazol-1-ium
- les sels du 1,4-diéthyl-5-[(4-{éthyl[3-(4-aza-1-azoniabicyclo[2.2.2]octan-1-yl)propyl]amino}phényl)diazényl]-4H-1,2,4-triazol-1-ium
- les sels du 1,4-diéthyl-5-[(4-{propyl[3-(4-aza-1-azoniabicyclo[2.2.2]octan-1-yl)propyl]amino}phényl)diazényl]-4H-1,2,4-triazol-1-ium
- les sels du 1,4-diallyl-5-[(4-{méthyl[3-(4-aza-1-azoniabicyclo[2.2.2]octan-1-yl)propyl]amino}phényl)diazényl]-4H-1,2,4-triazol-1-ium
- les sels du 1,4-diallyl-5-[(4-{éthyl[3-(4-aza-1-azoniabicyclo[2.2.2]octan-1-yl)propyl]amino}phényldiazényl]-4H-1,2,4-triazol-1-ium
- les sels du 1,4-diallyl-5-[(4-{propyl[3-(4-aza-1-azoniabicyclo[2.2.2]octan-1-yl)propyl]amino}phényl)diazényl]-4H-1)2,4-triazol-1-ium
- les sels du 1,4-dibenzyl-5-[(4-{méthyl[3-(4-aza-1-azoniabicyclo[2.2.2]octan-1-yl)propyl]amino}phényl)diazényl]-4H-1,2,4-triazol-1-ium
- les sels du 1,4-dibenzyl-5-[(4-{éthyl[3-(4-aza-1-azoniabicyclo[2.2.2]octan-1-yl)propyl]amino}phényl)diazényl]-4H-1,2,4-triazol-1-ium
- les sels du 1,4-dibenzyl-5-[(4-{propyl[3-(4-aza-1-azoniabicyclo[2.2.2]octan-1-yl)propyl]amino}phényl)diazényl]-4H-1(2(4-triazol-1-ium
- les sels du 1,3-diméthyl-2-[(4-{méthyl[2-(4-aza-1-azoniabicyclo[2.2.2]octan-1-yl)éthyl]amino}phényl)diazényl]-1H-imidazol-3-ium
- les sels du 1,3-diméthyl-2-[(4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(4-{propyl[2-(4-aza-1-azoniabicyclo[2.2.2]octan-1-yl)éthyl]amino}phényl)diazényl]-1H-imidazol-3-ium
- les sels du 1,3-diéthyl-2-[(4-{méthyl[2-(4-aza-1-azoniabicyclo[2.2.2]octan-1-yl)éthyl]amino}phényl)diazényl]-1H-imidazol-3-ium
- les sels du 1,3-diéthyl-2-[(4-{éthyl[2-(4-aza-1-azoniabicyclo[2.2.2]octan-1-yl)éthyl]amino}phényl)diazényl]-1H-imidazol-3-ium
- les sels du 1,3-diéthyl-2-[(4-{propyl[2-(4-aza-1-azoniabicyclo[2.2.2]octan-1-yl)éthyl]amino}phényl)diazényl]-1H-imidazol-3-ium
- les sels du 1,3-diallyl-2-[(4-{méthyl[2-(4-aza-1-azoniabicyclo[2.2.2]octan-1-yl)éthyl]amino}phényl)diazényl]-1H-imidazol-3-ium
- les sels du 1,3-diallyl-2-[(4-{éthyl[2-(4-aza-1-azoniabicyclo[2.2.2]odan-1-yl)éthyl]amino}phényl)diazényl]-1H-imidazol-3-ium
- les sels du 1,3-diallyl-2-[(4-{propyl[2-(4-aza-1-azoniabicyclo[2.2.2]octan-1-yl)éthyl]amino}phényl)diazényl]-1H-imidazol-3-ium
- les sels du 1,3-dibenzyl-2-[(4-{méthyl[2-(4-aza-1-azoniabicyclo[2.2.2]octan-1-yl)éthyl]amino}phényl)diazényl]-1H-imidazol-3-ium
- les sels du 1,3-dibenzyl-2-[(4-{éthyl[2-(4-aza-1-azoniabicyclo[2.2.2]octan-1-yl)éthyl]amino}phényl)diazényl]-1H-imidazol-3-ium
- les sels du 1,3-dibenzyl-2-[(4-{propyl[2-(4-aza-1-azoniabicyclo[2.2.2]octan-1-yl)éthyl]amino}phényl)diazényl]-1H-imidazol-3-ium
- les sels du 1,3-diméthyl-2-[(2-méthyl-4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(2-méthoxy-4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(2-chloro-4-{éthyl[2-(4-aza-1-azoniabicyclo[2.2.2]octan-1-yl)éthyl]amino}phényl)diazényl]-1H-imidazol-3-ium
- les sels du 1,3-diméthyl-2-[(4-{méthyl[3-(4-aza-1-azoniabicyclo[2.2.2]octan-1-yl)propyl]amino}phényl)diazényl]-1H-imidazol-3-ium
- les sels du 1,3-diméthyl-2-[(4-{éthyl[3-(4-aza-1-azoniabicyclo[2.2.2]octan-1-yl)propyl]amino}phényl)diazényl]-1H-imidazol-3-ium
- les sels du 1,3-diméthyl-2-[(4-{propyl[3-(4-aza-1-azoniabicyclo[2.2.2]octan-1-yl)propyl]amino}phényl)diazényl]-1H-imidazol-3-ium
- les sels du 1,3-diéthyl-2-[(4-{méthyl[3-(4-aza-1-azoniabicyclo[2.2.2]octan-1-yl)propyl]amino}phényl)diazényl]-1H-imidazol-3-ium
- les sels du 1,3-diéthyl-2-[(4-{éthyl[3-(4-aza-1-azoniabicyclo[2.2.2]octan-1-yl)propyl]amino}phényl)diazényl]-1H-imidazol-3-ium
- les sels du 1,3-diéthyl-2-[(4-{propyl[3-(4-aza-1-azoniabicyclo[2.2.2]octan-1-yl)propyl]amino}phényl)diazényl]-1H-imidazol-3-ium
- les sels du 1,3-diallyl-2-[(4-{méthyl[3-(4-aza-1-azoniabicyclo[2.2.2]octan-1-yl)propyl]amino}phényl)diazényl]-1H-imidazol-3-ium
- les sels du 1,3-diailyl-2-[(4-{éthyl[3-(4-aza-1-azoniabicyclo[2.2.2]octan-1-yl)propyl]amino}phényl)diazényl]-1H-imidazol-3-ium
- les sels du 1,3-diallyl-2-[(4-{propyl[3-(4-aza-1-azoniabicyclo[2-2.2]octan-1-yl)propyl]amino}phényl)diazényl]-1H-imidazol-3-ium
- les sels du 1,3-dibenzyl-2-[(4-{méthyl[3-(4-aza-1-azoniabicyclo[2.2.2]octan-1-yl)propyl]amino}phényl)diazényl]-1H-imidazol-3-ium
- les sels du 1,3-dibenzyl-2-[(4-{éthyl[3-(4-aza-1-azoniabicyclo[2.2.2]octan-1-yl)propyl]amino}phényl)diazényl]-1H-imidazol-3-ium
- les sels du 1,3-dibenzyl-2-[(4-{propyl[3-(4-aza-1 -azoniabicyclo[2.2.2]octan-1-yl)propyl]amino}phényl)diazényl]-1H-imidazol-3-ium
